# EUROPEAN PATENT SPECIFICATION

(11) **EP 1 420 800 B1**
(45) Date of publication and mention of the grant of the patent: **28.09.2011**
(21) Application number: 02747109.3
(22) Date of filing: 02.08.2002
(51) Int. Cl.: A61K 31/713, A61K 38/11, A61K 38/55, A61P 3/00, A61P 5/10, A61P 25/00, A61P 25/28, A61P 25/30, G01N 33/50

(54) **MODULATION OF INSULIN-REGULATED AMINOPEPTIDASE (IRAP)/ANGIOTENSIN IV (AT 4) RECEPTOR ACTIVITY**
MODULIERUNG DER INSULIN-GEREGELTEN AMINOPEPTIDASE (IRAP)/ANGIOTENSIN IV (AT-4) REZEPTORAKTIVITÄT
MODULATION DE L'ACTIVITE DU RECEPTEUR (AT 4) D'AMINOPEPTIDASE (IRAP)/D'ANGIOTENSINE IV REGULEE PAR L'INSULINE

(30) Priority: 02.08.2001 AU PR677201; 17.10.2001 US 330170 P
(43) Date of publication of application: 26.05.2004
(73) Proprietor: HOWARD FLOREY INSTITUTE OF EXPERIMENTAL PHYSIOLOGY AND MEDICINE, Parkville VIC 3052 (AU)
(72) Inventor: ALBISTON, Anthony, L, East Brunswick, Victoria 3057 (AU); MCDOWALL, Sharon, G, Coburg, Victoria 3058 (AU); MENDELSOHN, Frederick, A. O., Carlton, Victoria 3053 (AU); CHAI, Siew, Yeen, Burwood East, Victoria 3151 (AU)
(74) Representative: Webber, Philip Michael
(86) International application number: PCT/AU2002/001034
(87) International publication number: WO 2003/011304

(56) References cited:
- EP-A1- 0 838 471
- EP-A1- 0 882 699
- WO-A-00/65340
- WO-A-01/96867
- WO-A-96/09317
- WO-A1-00/77225
- WO-A1-94/00492
- WO-A1-98/01465
- WO-A2-00/12544
- LEE JOOHYUNG ET AL: "Potentiation of cholinergic transmission in the rat hippocampus by angiotensin IV and LVV-hemorphin-7" NEUROPHARMACOLOGY, vol. 40, no. 4, March 2001 (2001-03), pages 618-623, XP002441139
- MOELLER INGRID ET AL: "The globin fragment LVV-hemorphin-7 is an endogenous ligand for the AT-4 receptor in the brain" JOURNAL OF NEUROCHEMISTRY, vol. 68, no. 6, 1997, pages 2530-2537, XP002441140
- GHOSH R N ET AL: "CELL-BASED, HIGH-CONTENT SCREEN FOR RECEPTOR INTERNALIZATION, RECYCLING AND INTRACELLULAR TRAFFICKING" BIOTECHNIQUES, vol. 29, no. 1, 2000, pages 170-175, XP001205387
- CHI NAI-WEN ET AL: "Tankyrase is a Golgi-associated mitogen-activated protein kinase substrate that interacts with IRAP in GLUT4 vesicles" JOURNAL OF BIOLOGICAL CHEMISTRY, vol. 275, no. 49, 2000, pages 38437-38444, XP002459426
- KAIN S R: "Green fluorescent protein (GFP): Applications in cell-based assays for drug discovery" DDT - DRUG DISCOVERY TODAY, vol. 4, no. 7, 1999, pages 304-312, XP002408383
- DATABASE WPI Week 200147, Derwent Publications Ltd., London, GB; Class B04, AN 2001-441674, XP008093263 & WO 01 46413 A1 (TAKEDA CHEMICAL INDUSTRIES) 28 June 2001
- DATABASE WPI Week 200231, Derwent Publications Ltd., London, GB; Class B04, AN 2002-269344, XP008093262 & WO 02 16428 A1 (TAKEDA CHEMICAL INDUSTRIES) 28 February 2002
- ALBISTON ET AL.: 'Evidence that the angiotensin IV (AT4) receptor is the enyzme insulin-regulated aminopeptidase' THE JOURNAL OF BIOLOGICAL CHEMISTRY vol. 272, no. 52, 28 December 2001, pages 48623 - 48626, XP008079923
- HERBST ET AL.: 'Insulin stimulates cell surface aminopeptidase activity toward vasopressin in adipocytes' AMERICAN JOURNAL OF PHYSIOLOGY vol. 272, no. 4, 1997, pages E600 - E606, XP008079937
- WRIGHT ET AL.: 'Contributions of the brain angiotensin IV-AT4 receptor subtype system to spatial learning' THE JOURNAL OF NEUROSCIENCE vol. 19, no. 10, 15 May 1999, pages 3952 - 3961, XP008079922
- 'Actions of newly discovered angiotensin peptides and receptors in brain', [Online] 05 May 1999, Retrieved from the Internet: <URL:http://web.archive.org/web/19990505225 323/http://www.hfi.unimelb.edu.au/research/ neurochemistry/neurochemistry_detail.html> [retrieved on 2002-09-10]
- RASMUSSEN ET AL.: 'Structure of the human oxytocinase/insulin-regulated aminopeptidase gene and localization to chromosome 5q21' EUROPEAN JOURNAL OF BIOCHEMISTRY vol. 267, 2000, pages 2297 - 2306, XP002978919
- MIESFIELD R.L.: 'Applied molecular genetics', 1999, WILEY-LISS INC., ISBN 0-471-15676-0 article 'Antisense genetics', pages 181 - 183, XP008079938

### SEQUENCE LISTING

<110> Howard Florey Institute of Experimental Physiology and Medicine
<120> MODULATION OF AMINOPEPTIDASE ACTIVITY
<130> P42903
<140> Australian PR6722
   <141> 2001-08-02
<160> 23
<170> PatentIn version 3.1
<210> 1
   <211> 19
   <212> PRT
   <213> Bos taurus
<400> 1
Thr Phe Arg
<210> 2
   <211> 6
   <212> PRT
   <213> Homo sapiens
<400> 2
   Val Tyr Ile His Pro Phe
   1               5
<210> 3
   <211> 10
   <212> PRT
   <213> Ovis aries
<400> 3
   Leu Val Val Tyr Pro Trp Thr Gln Arg Phe
   1               5                   10
<210> 4
   <211> 8
   <212> PRT
   <213> Ovis aries
   <400> 4
   Lys Tyr Leu Pro Gly Pro Leu Gln
   1               5
<210> 5
   <211> 6
   <212> PRT
   <213> artificial sequence
<220>
   <223> Nle substitution at position 1
<220>
   <221> MOD_RES
   <222> (1)..(1)
   <223> Nle
<400> 5
   Leu Tyr Ile His Pro Phe
   1 5
<210> 6
   <211> 31
   <212> DNA
   <213> artificial sequence
<220>
   <223> antisense oligonucleotide from mouse IRAP cDNA sequence
<400> 6
   gaggttaggt ttggatgtag gctaagttca t 31
<210> 7
   <211> 28
   <212> DNA
   <213> artificial sequence
<220>
   <223> antisense oligonucleotide from mouse IRAP cDNA sequence
<400> 7
   attgatcagg ttggctcggt ctttgtca 28
<210> 8
   <211> 28
   <212> DNA
   <213> artificial sequence
<220>
   <223> Probe for both splice variants
<400> 8
   ccaagtaagt gctcatcttc acactctc 28
<210> 9
   <211> 31
   <212> DNA
   <213> artificial sequence
<220>
   <223> Probe for both splice variants of AT4 receptor/IRAP
<400> 9
   gtgttactgt catacagaag tgtctcctct c 31
<210> 10
   <211> 28
   <212> DNA
   <213> artificial sequence
<220>
   <223> Probe for both splice variants
<400> 10
   cagtttcatg gcagtagtag agcagttc 28
<210> 11
   <211> 33
   <212> DNA
   <213> artificial sequence
<220>
   <223> Probe for AT4 receptor/IRAP
<400> 11
   cattggtgaa gggctccatt tccttacagc cgg 33
<210> 12
   <211> 37
   <212> DNA
   <213> artificial sequence
<220>
   <223> Probe specific for splice variant of AT4 receptor/IRAP
<400> 12
   cttccctcaa acatgctgtt ttcaatcata ttcctgg 37
<210> 13
   <211> 24
   <212> DNA
   <213> artificial sequence
<220>
   <223> Probe specific for splice variant of AT4 receptor/IRAP
<400> 13
   tggaggagga tgaagaggat tatg 24
<210> 14
   <211> 26
   <212> DNA
   <213> artificial sequence
<220>
   <223> Probe for AT4 receptor/IRAP
<400> 14
   ctacgctatg aactcagcct acaccc 26
<210> 15
   <211> 27
   <212> DNA
   <213> artificial sequences
<220>
   <223> Probe for AT4 receptor/IRAP
<400> 15
   gcctacaccc gaacctaacc tcgatga 27
<210> 16
   <211> 24
   <212> DNA
   <213> artificial sequence
<220>
   <223> Probe for AT4 receptor/IRAP
<400> 16
   cacaggtcat aatatttcaa gagt 24
<210> 17
   <211> 26
   <212> DNA
   <213> artificial sequence
<220>
   <223> Probe for AT4 receptor/IRAP
<400> 17
   aagccaagaa aaacaagctg agatcc 26
<210> 18
   <211> 18
   <212> DNA
   <213> artificial sequence
<220>
   <223> Probe for AT4 receptor/IRAP
<400> 18
   ataagggatg agcaatac 18
<210> 19
   <211> 25
   <212> DNA
   <213> artificial sequence
<220>
   <223> Probe for AT4 receptor/IRAP
<400> 19
   caccgcttta tcaaatatgc ctaag 25
<210> 20
   <211> 25
   <212> DNA
   <213> artificial sequence
<220>
   <223> Probe for AT4 receptor/IRAP
<400> 20
   atatgcctaa gaagtcatca gtcgt 25
<210> 21
   <211> 25
   <212> DNA
   <213> artificial sequence
<220>
<223> Probe for AT4 receptor/IRAP
<400> 21
   ctactttgaa attcagtacc cactt 25
<210> 22
   <211> 26
   <212> DNA
   <213> artificial sequence
<220>
   <223> Probe for AT4 receptor/IRAP
<400> 22
   ttcagtaccc acttaagaaa ttggat 26
<210> 23
   <211> 25
   <212> DNA
   <213> artificial sequence
<220>
   <223> Probe for AT4 receptor/IRAP
<400> 23
   tcaccttccg agaggagaca cttct 25

## Description

### FIELD OF THE INVENTION

This invention relates to methods of identifying modulators of insulin-regulated aminopeptidase activity. These modulators have the ability to increase local concentration of a wide range of peptide hormones, thereby enhancing the activity of these hormones. The modulators may enhance learning and memory in both normal subjects and those with memory disorders. They also stimulate cellular proliferation while reducing apoptosis, thus stimulating tissue growth development, and affect angiogenesis. Thus they are also useful in tissue repair and regeneration.

### BACKGROUND OF THE INVENTION

There are a number of conditions which have no known treatment, or for which the treatments that are available are not effective. For example, disorders of the central nervous system, including Alzheimer's disease (AD) and other forms of dementia and memory loss, motor neurone diseases, disorders of the cardiovascular system, including cardiac hypertrophy, congestive heart failure, hypertension, and atherosclerosis, and disorders of development and growth, including disorders of glucose and fat metabolism, are all serious conditions for which effective treatment is currently unavailable. Given the importance of these conditions, it is readily apparent that effective treatments are urgently needed.

Alzheimer's disease (AD), for example, is one of the major health care problems facing first world countries. In 1995, 130,000 Australians aged over 65 years had moderate to severe dementia, and it is estimated that by 2041 the number of people with dementia in Australia will have increased by 254% (Henderson and Jorm, 1998). Despite a dramatic increase in the understanding of the neuropathogenic and neurochemical alterations accompanying AD, physicians have as yet no pharmacological agent that can be prescribed safely, either to arrest or reverse this condition.

Some of the other conditions identified above are also increasing in prevalence, and will become increasingly economically and socially burdensome. Accordingly, there is a need for new forms of treatment and new therapeutic agents which can provide sufferers of these diseases with effective treatment.

Angiotensin IV (Ang IV) is a hexapeptide with the sequence VYIHPF (SEQ ID NO:1), which has been shown *in vitro* to be produced by the consecutive actions of aminopeptidases A and N on angiotensin II. This hexapeptide was initially thought to be inactive, but has subsequently been shown to elicit actions in the brain and blood vessels. Ang IV acts via a specific binding site, designated the Angiotensin IV receptor (AT₄ receptor).

The AT₄ receptor is defined as a high affinity binding site which selectively binds Ang IV with an affinity ranging from 1-10 nM (Swanson et al, 1992). The AT₄ receptor isolated from bovine adrenal membranes is a glycoprotein of 165 kDa, with a single transmembrane domain, a large extracellular domain, and a small intracellular domain (Zhang et al, 1998). In the brain, the AT₄ receptor occurs as a 150 kDa glycoprotein (Zhang et al, 1999). We have shown that a decapeptide, LVVYPWTQRF (SEQ ID NO:2) (Moeller et al, 1996) binds with nanomolar affinity to the AT₄ receptor, and can mimic the actions of Ang IV, and that Nle'-Ang IV (Nle'-YIHPF) (SEQ ID NO:4) binds with sub-nanomolar affinity to AT₄ receptor (Zhang et al, 1999).

AT₄ receptors are widely distributed in the brains of guinea-pig (Miller-Wing et al, 1993), rat (Roberts et al, 1995), sheep (Moeller et al, 1996), monkey (Moeller et al, 1996), and human (Chai et al, 2000), and the distributions of the receptors are highly conserved throughout these species. The receptor sites occur in high abundance in the basal nucleus of Meynert, in the CA2 and dentate gyrus of the hippocampus, and throughout the neocortex, a distribution which closely resembles those of cholinergic neurones and their projections, and is consistent with the memory-enhancing effects of the receptor. High levels of the receptors are also found in most brain regions involved in motor control, including the motor cortex, ventral lateral thalamic nucleus, cerebellum and the cranial nerve motor nuclei. Many sensory regions also contain moderate levels of the AT₄ receptors, suggesting a more widespread role for this receptor in the brain.

Central infusions of Ang IV, Nle-Ang IV and Norleucinal Ang IV, which are agonists of the AT₄ receptor, were found to facilitate memory retention and retrieval in rats in the passive avoidance and Morris water maze paradigms (Braszko et al, 1988, Wright et al, 1993, Wright et al, 1999). In two rat models of amnesia, respectively induced by scopolamine or perforant pathway lesion, the AT₄ receptor agonists reversed the performance deficits detected in the Morris water maze paradigm (Pederson et al, 1998; Wright et al, 1999). Ang IV was also reported to improve long-term memory in crabs (Delorenzi et al, 1999). WO 94/00492 discloses angiotensin AT4 receptor and AIV ligand system for binding a small N-terminal hexapeptide fragment of Angiotensin II (referred to as AIV, with amino acid sequence Val1-Tyr2-Ile3-His4-Pro5-Phe6). Also disclosed are processes for isolating angiotensin AT4 receptor and AIV angiotensinase, identifying angiotensin AIV agonists and antagonists, and constructing diagnostic assays to specifically measure AIV and AI-specific angiotensinase in biological fluids. Insulin-regulated aminopeptidase (IRAP) is a constituent of vesicles containing the glucose transporter molecule GLUT4. See U.S. Patent No. 5,968,784 and No. 5,972,600, both by Knowles et al. It is known that the distribution and recycling of both IRAP and GLUT4 between cellular vesicles and the cell membrane is regulated by insulin signalling, and thereby modulates glucose, fat and energy metabolism in tissues such as muscle, adipose tissue, and cardiac tissue (Waters et al, 1997, Ross et al, 1998, Garza and Birnbaum, 2000). However, it has never to our knowledge previously been suggested that IRAP might have any role in central nervous system function, and more specifically it has not been suggested that this protein has any role in memory or learning.

We have now isolated the AT₄ receptor by protein purification, and have determined its amino acid sequence. We surprisingly discovered that the AT₄ receptor has a span of 18 amino acid residues which has a 95% sequence homology with the deduced amino acid sequence of human oxytocinase (TrEMBL accession # 000769), which is known to be a homologue of insulin-regulated aminopeptidase (IRAP) (Rasmussen et al, 2000). This enabled us to demonstrate that IRAP is the AT₄ receptor, and to hypothesise that modulation of the activity of IRAP by peptides such as Ang IV, Nle¹-Ang IV, LVVYPWTQRF (SEQ ID NO:2) and KYLPGPLQ (SEQ ID NO:3) will activate neurones in the central nervous system to enhance learning and memory. Moreover, the identification of IRAP as the AT₄ receptor means that screening assays can be developed to identify compounds which have the ability to modulate IRAP enzymatic activity. Such agents can be used to increase serum and tissue levels of biologically active compounds such as arginine-vasopressin, oxytocin, somatostatin, angiotensin III, Lys-bradykinin, neurotensin, met-enkephalin, dynorphin A, neurokinin A, neuromedin B, cholecystokinin 8, insulin, insulin-like growth factor 1 or insulin-like growth factor 2 which may assist in preventing or treating conditions associated with deficient activity of these compounds.

### SUMMARY OF THE INVENTION

The invention provides a method as claimed in claim 1. DISCLOSED METHODS Also disclosed is a method of *in vitro* screening for compounds which have the ability to modulate the activity of AT₄ receptor/IRAP, comprising the steps of
(a) assessing the ability of a candidate compound to bind to purified or recombinant AT₄ receptor/IRAP protein, and one or more of
(b) assessing the ability of the compound to modulate the enzymatic activity of purified or recombinant AT₄ receptor/IRAP protein;
(c) assessing the effect of the compound on the interaction between purified or recombinant AT₄ receptor/IRAP protein and a protein which interacts with AT₄ receptor/IRAP;
(d) assessing the ability of the candidate compound to modulate one or more signalling or intracellular pathways activated by binding of the candidate compound to AT₄ receptor/IRAP;
(e) assessing the ability of the candidate compound to modulate an activity of purified or recombinant AT₄ receptor/IRAP protein;
(f) assessing the ability of the candidate compound to translocate AT₄ receptor/IRAP to the cell surface Also disclosed is a method which comprises the steps of assessing the ability of the candidate compound to
   (a) bind to purified or recombinant AT₄ receptor/IRAP protein or to a protein which interacts with AT₄ receptor/IRAP;
   (b) alter the ability of a protein to interact with AT₄ receptor/IRAP; and
   (c) modify the enzymatic or signalling activities of AT₄ receptor/IRAP.

Also disclosed are methods which may be used in identifying compounds which translocate AT₄ receptor/IRAP to the cell surface, the method comprising the steps of
(a) exposing cells of a stably transfected cell line expressing AT₄ receptor/IRAP to a candidate compound, and
(b) assessing the amount of AT₄ receptor/IRAP expressed on the cell surface.

In particular embodiments, the candidate compound may be an intracellular or extracellular protein, or the protein which interacts with AT₄ receptor/IRAP may be tankyrase.

In the claims which follow and in the description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

### BRIEF DESCRIPTION OF THE FIGURES

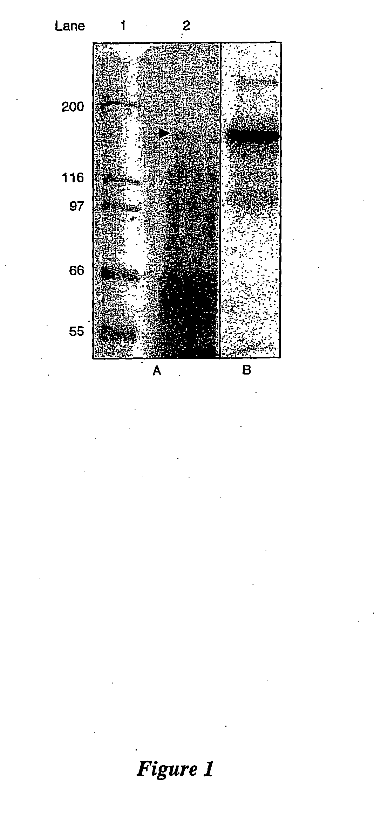

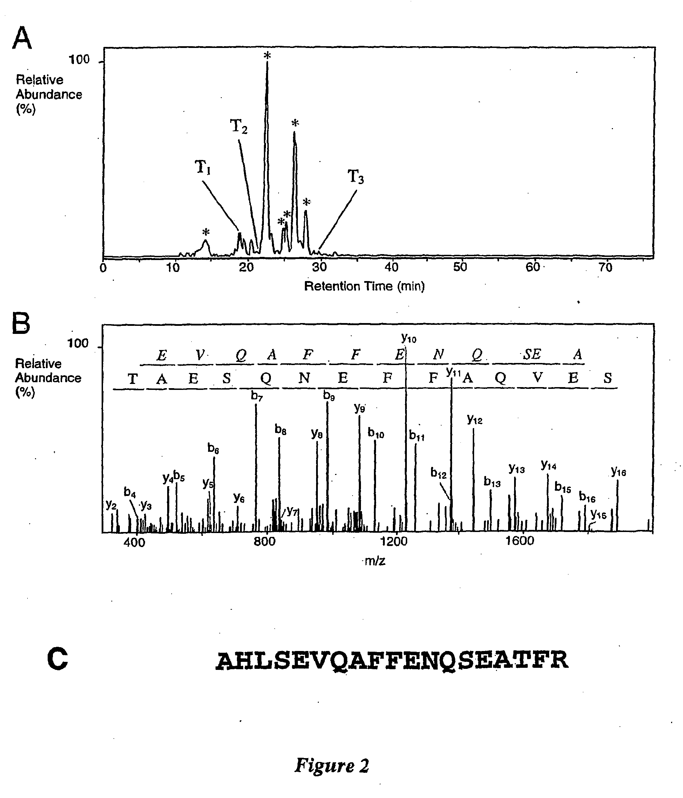

Figure 1 shows non-reducing SDS-PAGE analysis of fractions from DEAE-Sepharose ion exchange purification of the AT₄ receptor. The gel was silver stained (A) and subjected to autoradiography (B). Proteins from bovine adrenal membranes, solubilised with CHAPS, were fractionated on a DEAE-Sepharose fast flow column. A silver-stained protein band (indicated by an arrowhead), which co-migrated with the radiolabelled band, was detected in eluted fractions, as shown in lane 2. Molecular weight markers are shown in lane 1, with the sizes in kDa indicated to the left of the figure. Figure 2 shows the results of capillary column RP-HPLC/ESI-MS/MS analysis of the silver-stained protein band shown in Figure 1 at Mᵣ 165kD.
   (A) MS/MS total ion current (TIC) trace of the tryptic digest. The peptide mixture was applied to a 0.2 mm ID column and a 60 min linear gradient (flow rate 1.7µL/min) was used from 0-100% B, in which solvent A was 0.1% v/v aqueous TFA, and solvent B was 0.1% v/v aqueous TFA in 60% acetonitrile. Peptides were sequenced by collision-induced dissociation (CID) of their parent ions. Peaks labelled with an asterisk were identified as trypsin auto-digestion peptides. Peptides T₁ and T₂ were identified using the SEQUEST algorithm as residues 321-349 and 208-220 respectively, from Bos taurus CD34 (TrEMBL accession number Q9XS61).
   (B) Collision-induced dissociation MS/MS spectrum of the doubly-charged ion of tryptic peptide T₃ (*m*/*z* 1106.0). Peptide T₃ could not be identified using SEQUEST, and was interpreted using *de novo* methods, i.e., manual interpretation of the β- and γ-type product ion series (Roepstorff and Fohlman, 1984), as shown. The sequence tag VQAFFE was deduced first, and this was searched against the TrEMBL database using the Fasta program. This sequence tag was found to be homologous to human oxytocinase (TrEMBL accession number 000769). Further residues from human oxytocinase (residue numbers 979-996) were found to fit the MS/MS spectrum. (C) The sequence of the 19 amino acid peptide thus identified is shown. It differs from homologous region of human oxytocinase only in the N-terminal residue, which is a threonine in the human sequence.

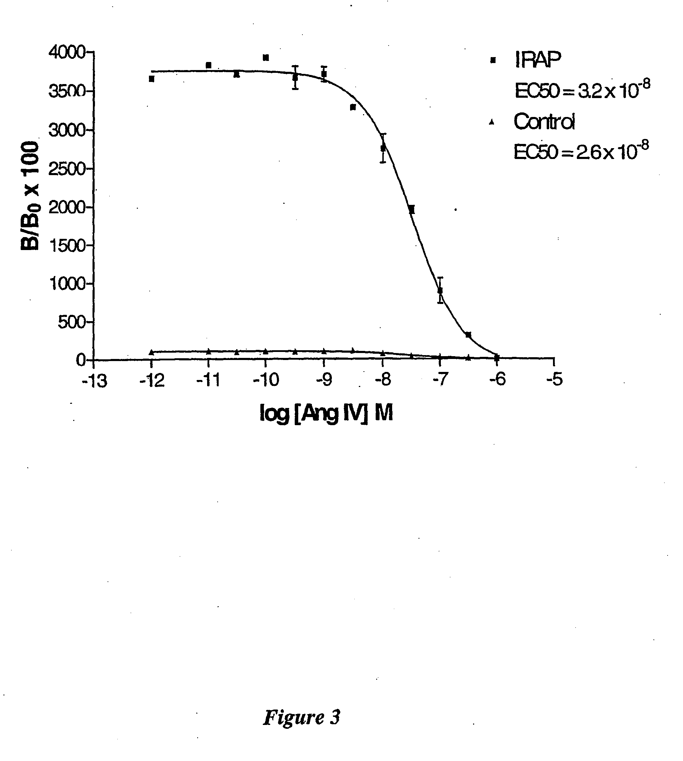

Figure 3 summarises results from two binding studies, showing an increase in ¹²⁵I-Nle¹ Ang IV binding to membranes prepared from HEK 293 cells which have been transfected with plasmids containing the IRAP cDNA (IRAP) compared to membranes from HEK 293 cells transfected with control plasmids (control). The binding of the radioligand is competitively inhibited by increasing concentrations of unlabelled Ang IV. Bo represents the total number of ¹²⁵I-Nle¹ Ang IV binding sites in the IRAP, transfected cells, and B/Bo x 100 represents the number of binding sites occupied, expressed as a percentage of the total number of sites in the IRAP transfected cells. Pci IRAP was prepared as described by Rogi et al, 1996.

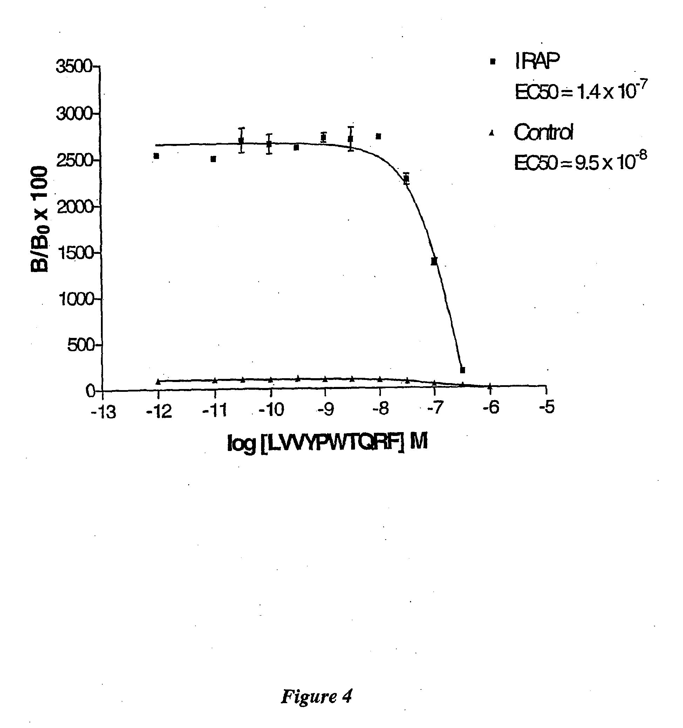

Figure 4 summarises results from a binding study showing an increase in ¹²⁵I-Nle¹ Ang IV binding to membranes prepared from HEK 293 cells transfected with plasmids containing the IRAP cDNA (IRAP), compared to membranes from HEK 293 cells transfected with control plasmids (control). The binding of the radioligand is competitively inhibited by increasing concentrations of unlabelled LVVYPWTQRF (SEQ ID NO:2), a known AT₄ receptor ligand. Bo represents the total number of ¹²⁵I-Nle¹ Ang IV binding sites in the IRAP transfected cells, and B/Bo x 100 represents the number of binding sites occupied, expressed as a percentage of the total number of sites in the IRAP transfected cells.

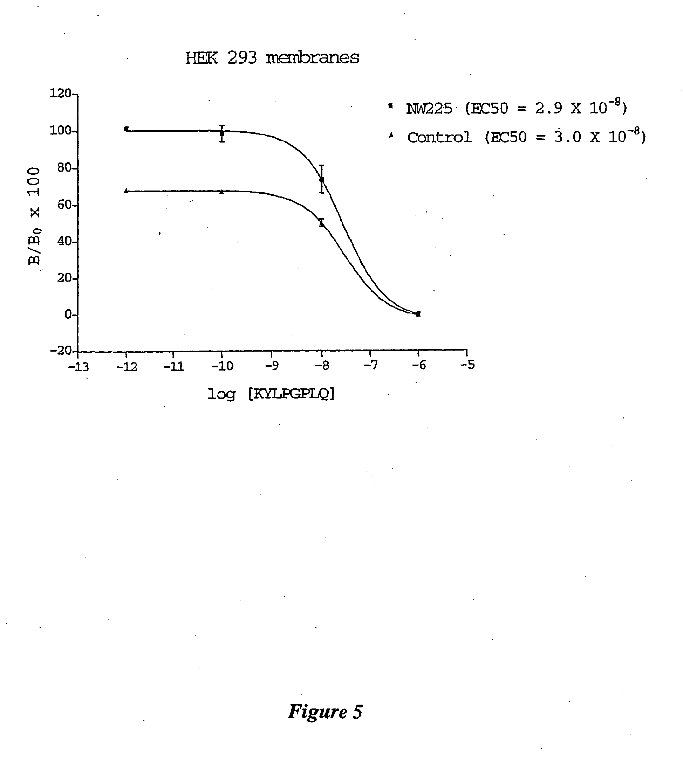

Figure 5 summarises results from a binding study showing an increase in ¹²⁵I-Nle¹ Ang IV binding to membranes prepared from HEK 293 cells transfected with plasmids containing the IRAP cDNA (IRAP) compared to membranes from HEK 293 cells transfected with control plasmids (control). The binding of the radioligand is competitively inhibited by increasing concentrations of unlabelled KYLPGPLQ (SEQ ID NO:3), a known AT₄ receptor ligand. Bo represents the total number of ¹²⁵I-Nle¹ Ang IV binding sites in the IRAP transfected cells and B/Bo x 100 represents the number of binding sites occupied, expressed as a percentage of the total number of sites in the IRAP transfected cells.

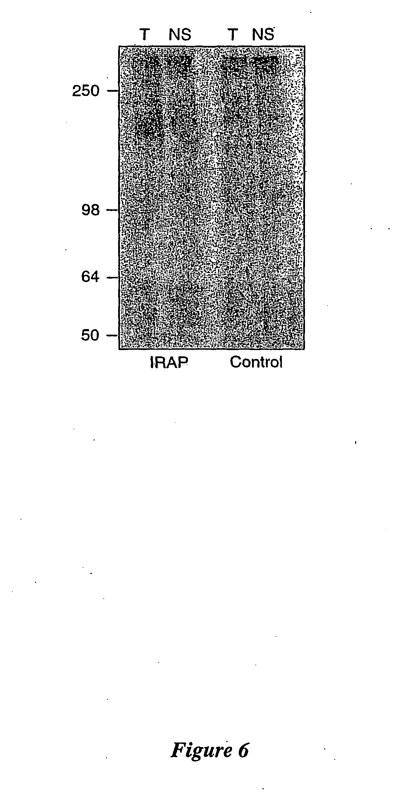

Figure 6 shows an autoradiograph of non-reducing SDS-PAGE analysis of [¹²⁵I] -Nle¹-BzPhe⁶-Gly⁷-Ang IV cross-linked AT₄ receptor/IRAPs from membranes from HEK 293-T cells transfected with IRAP cDNA (IRAP), compared to membranes from HEK 293-T cells transfected with control plasmids (control), showing total (T) and non-specific (NS) binding. Transfected cells were solubilised in 1% CHAPS, 50 mM Tris, pH 6.8, 5 mM EDTA for 4 h at 4°C. Membranes were removed by centrifugation, and the solubilised receptor incubated with 0.1 µCi [¹²¹I]-Nle¹-BzPhe⁶-Gly⁷-Ang IV, 20 µM bestatin, 100 µM PMSF, 2.5 mM phenanthroline, in a total volume of 100 µl for 90 min at 37°C, then UV irradiated at 365 nm for 2 h on ice. Two specific radiolabelled bands are visible in cells transfected with IRAP cDNA; a major band of approximately 165kDa and a minor band of >250 kDa.

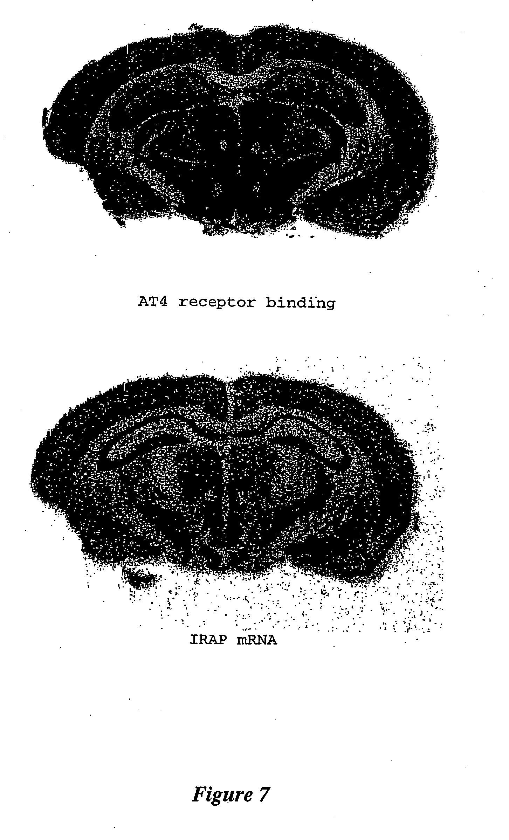

Figure 7 demonstrates the distribution of AT₄ receptor binding using ¹²⁵I-Nle Ang IV binding in a mouse brain section and IRAP mRNA by *in situ* hybridization histochemistry, in an adjacent section.

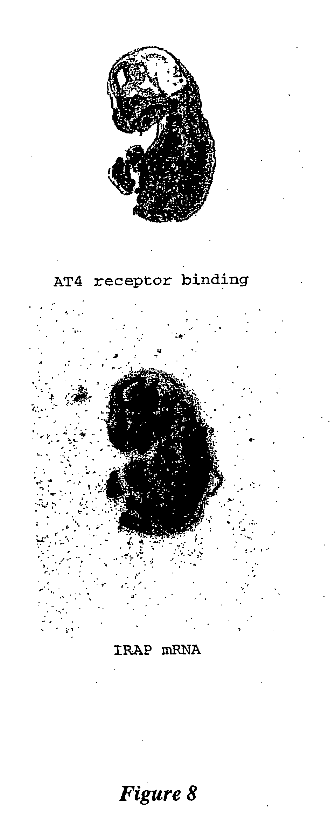

Figure 8 illustrates the distribution of AT₄ receptor binding in E15 mouse embryo using ¹²⁵I-Nle Ang IV and IRAP mRNA using antisense oligonucleotides.

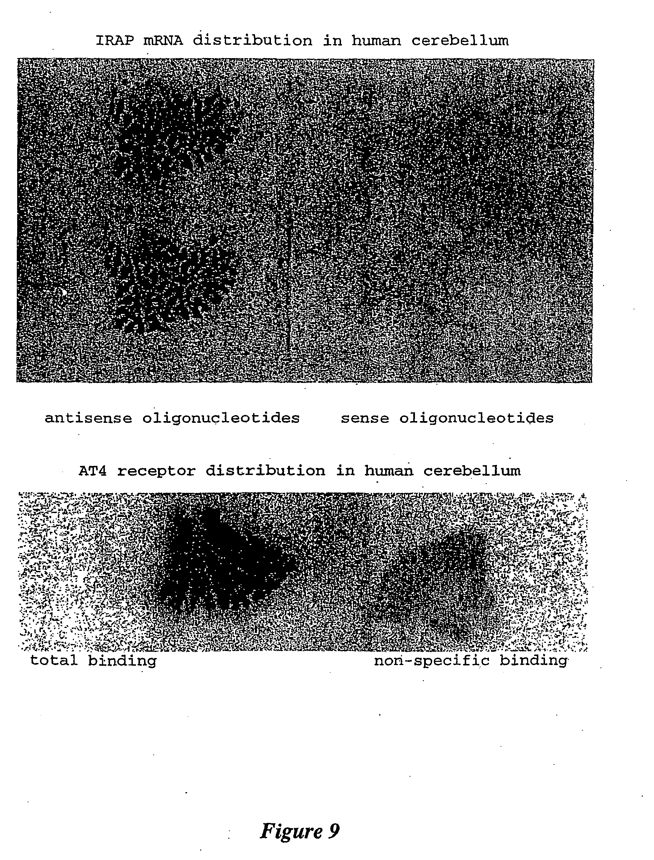

Figure 9 demonstrates the distribution of IRAP mRNA in human cerebellar sections by *in situ* hybridization histochemistry, using antisense and sense oligonucleotide sequences derived from the human IRAP cDNA sequence (Top panels). The antisense sequences used include nucleotides 1136-1109, nucleotides 1407-1377 and nucleotides 2587-2560 of the human IRAP sequence (Human Genbank Acc.U62768). The adjacent sections of the cerebellum were labelled with ¹²⁵I-Nle Ang IV to demonstrate AT₄ receptor binding (Bottom panel).

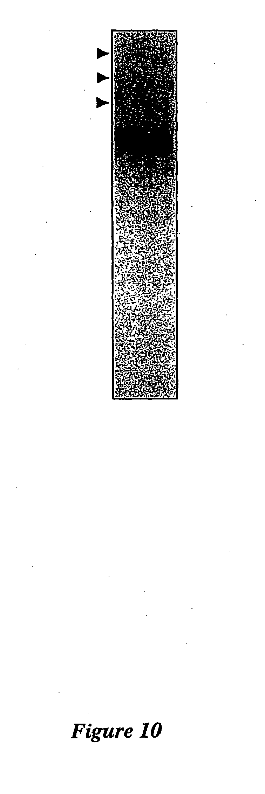

Figure 10 shows an autoradiograph of non-reducing SDS-PAGE analysis of [¹²⁵I] -Nle¹-Ble¹-BzPhe⁶-Gly⁷-Ang IV cross-linked AT₄ receptor/IRAPs from bovine adrenal membranes, purified by DEAE-Sepharose anion exchange chromatography. A major band with apparent molecular weight of 165 kDa is visible, as well as three higher molecular weight complexes (indicated by arrowheads).

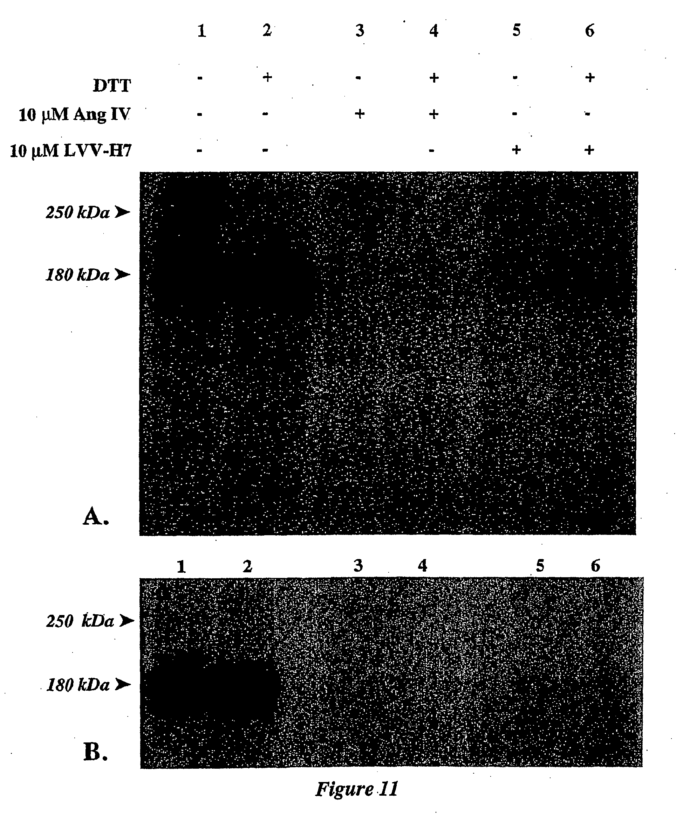

Figure 11 shows an autoradiograph of SDS-PAGE analysis of [¹²⁵I]-Nle¹-BzPhe⁶-Gly⁷-Ang.IV cross-linked AT₄ receptors from SK-N-MC cells (A) and bovine adrenal membranes (B). Shown are total [¹²⁵I] -Nle¹-BzPhe⁶-Gly⁷-Ang IV binding to SK-N-MC and bovine adrenal membranes under non-reducing conditions (*Lane 1*) and reducing conditions (*Lane 2*), in the presence of 10 µm unlabelled Ang IV under non-reducing (*Lane 3*) and reducing conditions (*Lane 4*) and in the presence of 10 µM unlabelled LVVYPWTQRF (SEQ ID NO:2) under non-reducing (*Lane 5*) and reducing conditions (*Lane 6*). A minor higher molecular weight radiolabelled complex, which migrates with an apparent molecular weight of approximately 250 kDA, is apparent under non-reducing conditions (*Lane 1*), but not under reducing conditions (*Lane 2*).

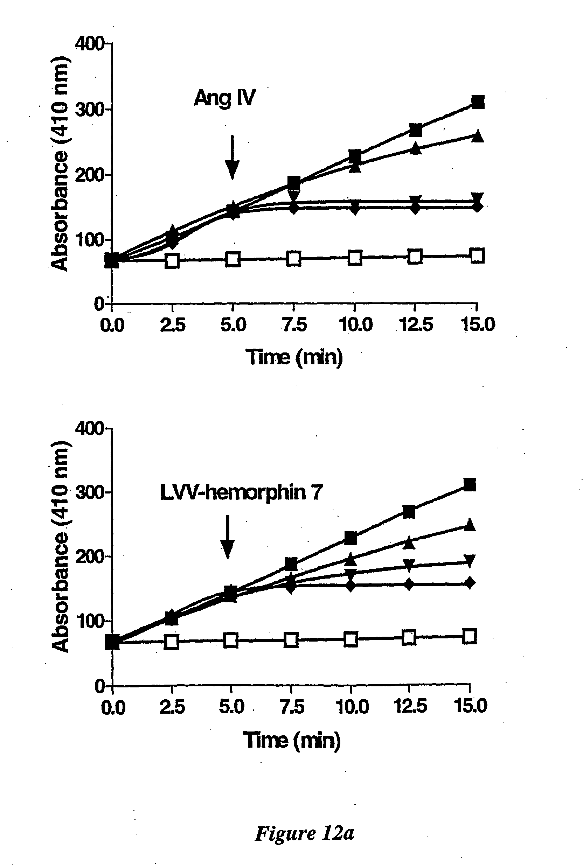

Figure 12a shows the results of measurement of inhibition of IRAP activity by Ang IV and LVVYPWTQRF (SEQ ID NO:2), using a fluorescence assay with the synthetic substrate L-leucyl-β-naphthylamide. The rate of hydrolysis of L-leucyl-β-naphthylamide by IRAP in the presence of 10 mM Ang IV or LVVYPWTQRF (SEQ ID NO:2) (LVV-hemorphin 7) was detected by the change in fluorescence at an excitation wavelength of 340 nm and an emission wavelength of 410 nm.

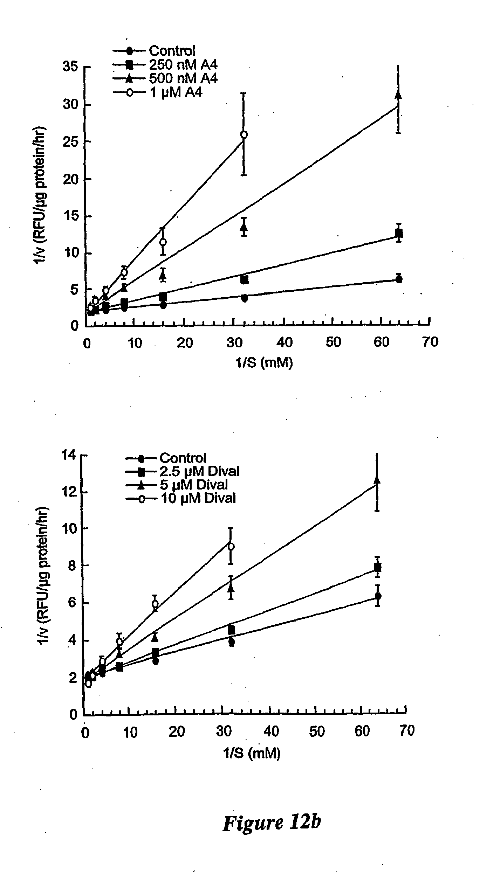

Figure 12b illustrates the kinetic analysis of IRAP inhibition. Double-reciprocal (Lineweaver-Burk) plots of IRAP activity in the absence and presence of three concentrations of Ang IV (Top panel) or divalinal Ang IV (Bottom panel) are shown.

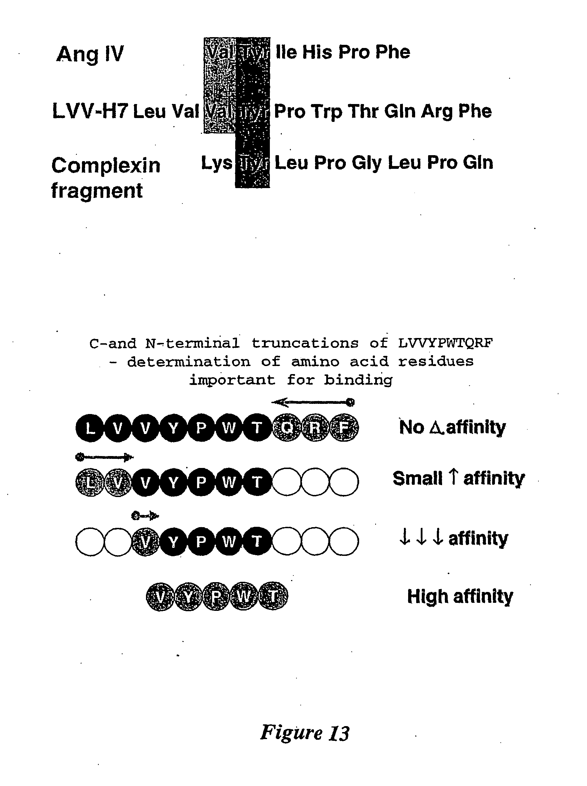

Figure 13 shows the amino acid residues of the three peptides which bind with high affinity to the AT₄ receptor/IRAP aligned at the tyrosine residue. Structure-activity studies on the longest peptide
   LVVYPWTQRF (SEQ ID NO:2)
   revealed that the residues VYPWT still retained full AT₄ receptor/IRAP binding activity.

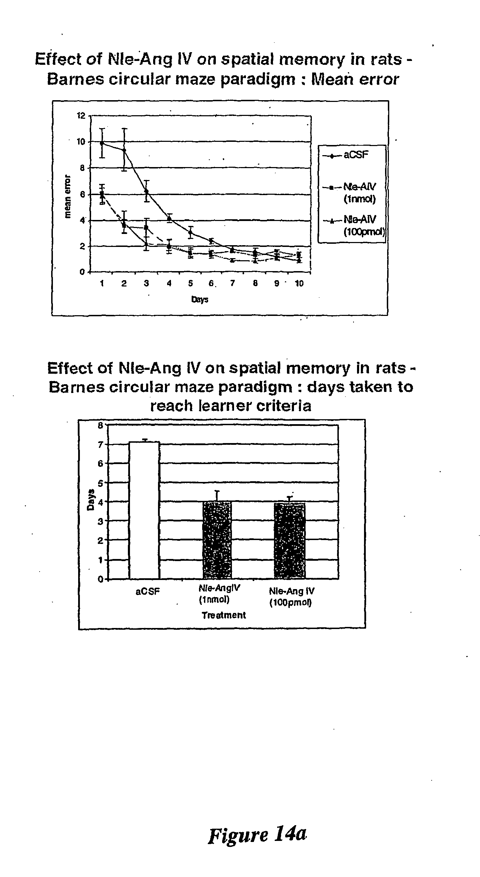

Figure 14a shows a summary of the effect of Nle Ang IV in the Barnes Maze spatial learning paradigm. The upper panel is a plot of the mean error of three treatment groups given artificial cerebrospinal fluid control, Nle Ang IV at 1 nmol or Nle Ang IV at 100 pmol respectively.. The lower panel is a plot of the number of days taken by the three treatment groups reach learner criteria.

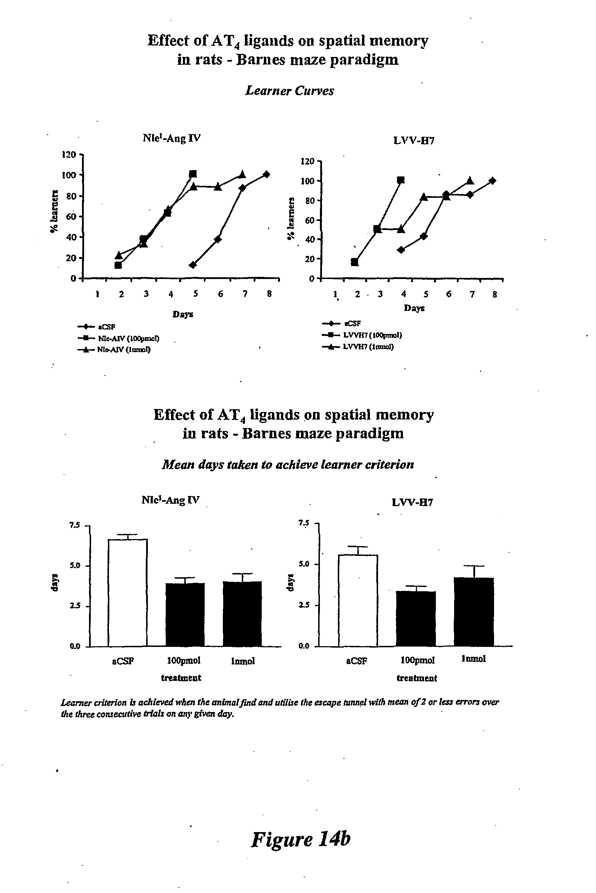

Figure 14b demonstrates the effect of AT₄ ligands, Nle-Ang IV and LVVYPWTQRF (SEQ ID NO:2), to accelerate learning in the Barnes circular maze paradigm. The top panel illustrates the learner curves, which are plots of the percentage of animals reaching learner criteria over the days of testing for the two peptide treatments. The bottom panel are plots of the mean number of days the animals take to reach learner criteria after a single dose of either peptide 5 min before the first trial on the first day.

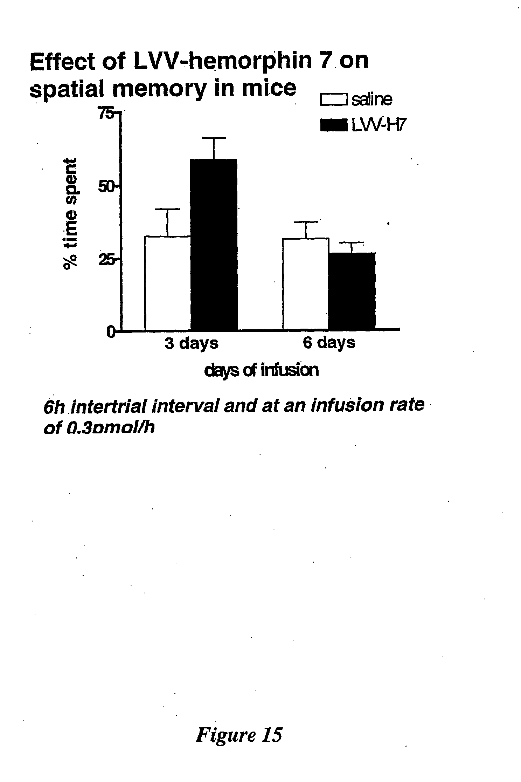

Figure 15 summarises the effect of chronic infusion of LVVYPWTQRF (SEQ ID NO:2) at a rate of 0.3 pmol/h on the Y maze spatial learning paradigm, after 3 and 6 days in mice. The plot is of the % time spent in the novel arm of the Y maze. Increased time spent in the novel arm reflects improved memory retention as a result of the treatment.

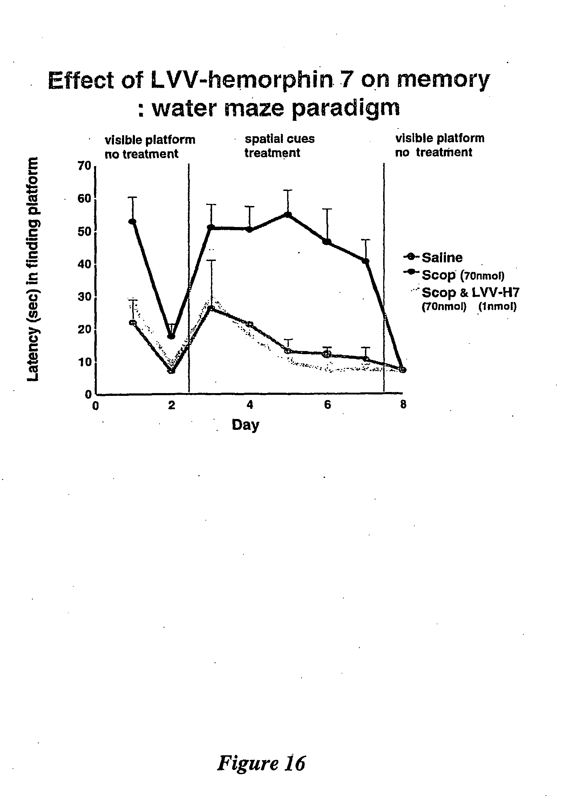

Figure 16 summarises the effect of an acute intracerebroventricular (icv) dose of LVVYPWTQRF (SEQ ID NO:2) on the amnesic effect of scopolamine treatment in the Morris Water Maze paradigm in rats. The latency in time in finding the submerged platform is a measure of the amnesic property of the different treatments.

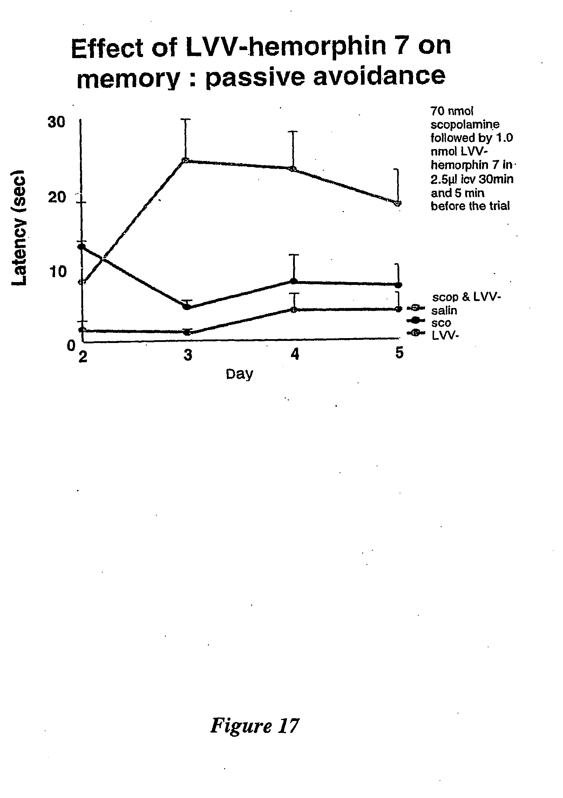

Figure 17 summarises the effect of an acute (icv) dose of LVVYPWTQRF (SEQ ID NO:2) on the amnesic effect of scopolamine treatment in the passive avoidance paradigm in rats. The latency in time in entering the dark chamber is a measure of the memory-enhancing property of the different treatments.

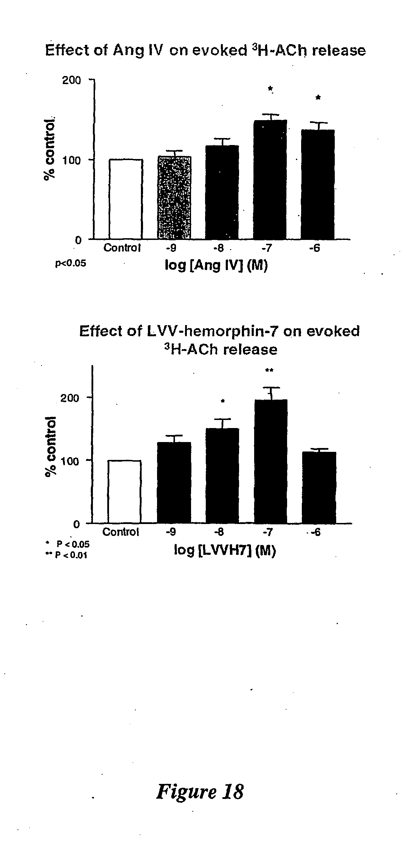

Figure 18 demonstrates the effect of Ang IV (A) and LVVYPWTQRF (SEQ ID NO:2) (B) on potassium-evoked ³H-choline release from rat hippocampal slices, using 10⁻⁹ to 10⁻⁶ M of each peptide. Values are the mean ± SEM of six experiments at each dose, and are expressed as a percentage of control. *p<0.05.

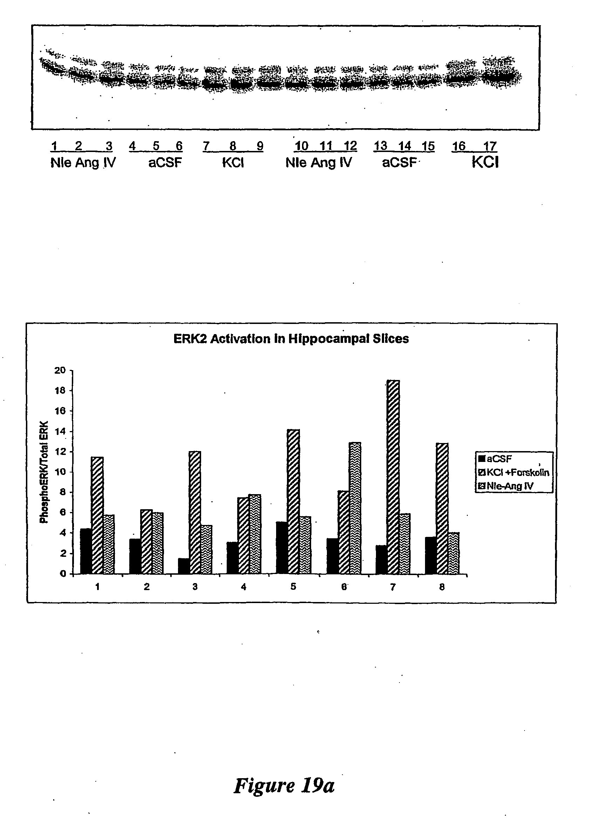

Figure 19a illustrates stimulation of ERK1 and ERK2 phosphorylation in rat hippocampal slices treated with 10⁻⁸ M Nle-Ang IV in aCSF, for 30 min, aCSF alone or 30 mM KCl and 5 mM forskolin for 10 min.

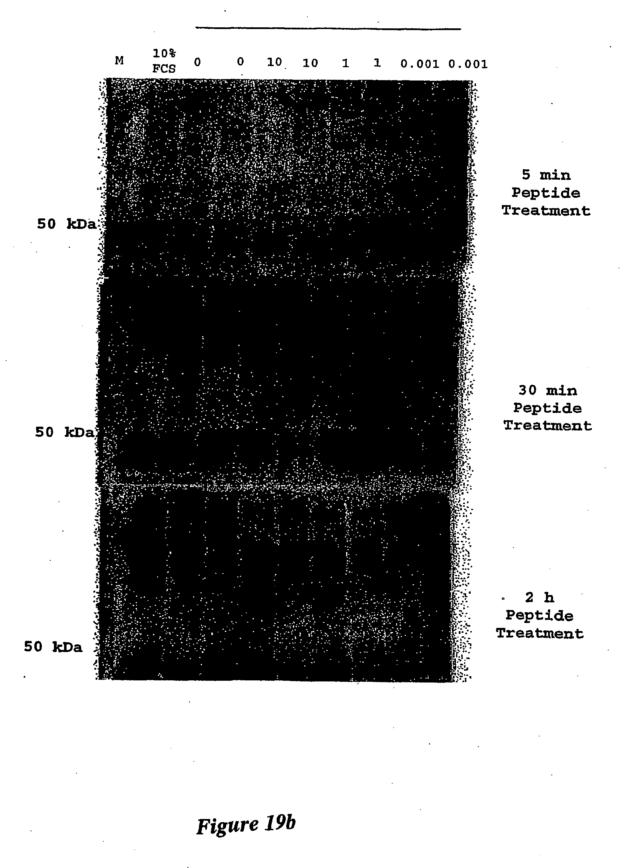

Figure 19b shows the effect of three different concentrations (0.001, 1 and 10 µM) of Nle-Ang IV on ERK1/ERK2 phosphorylation in HEK 293 cells transfected with human IRAP treated for 5 min, 30 min and 120 min with the peptide.

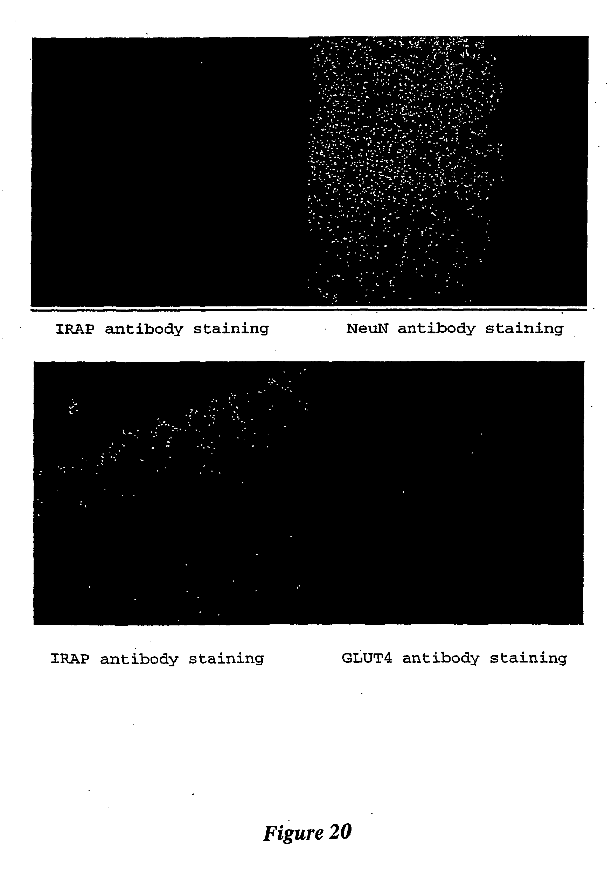

Figure 20 shows a section of mouse hippocampus stained with an antibody directed against the cytoplasmic tail of murine IRAP. The IRAP antibody staining was detected in the pyramidal cell layer of CA3 region of Ammon's horn (top panel - right), corresponding to neurones demonstrated by double labelling with the neuronal marker NeuN antibody (top panel - left). The bottom panel shows that IRAP (left) was found in the same neurones as GLUT4 (right) but not in the same sub-cellular compartments.

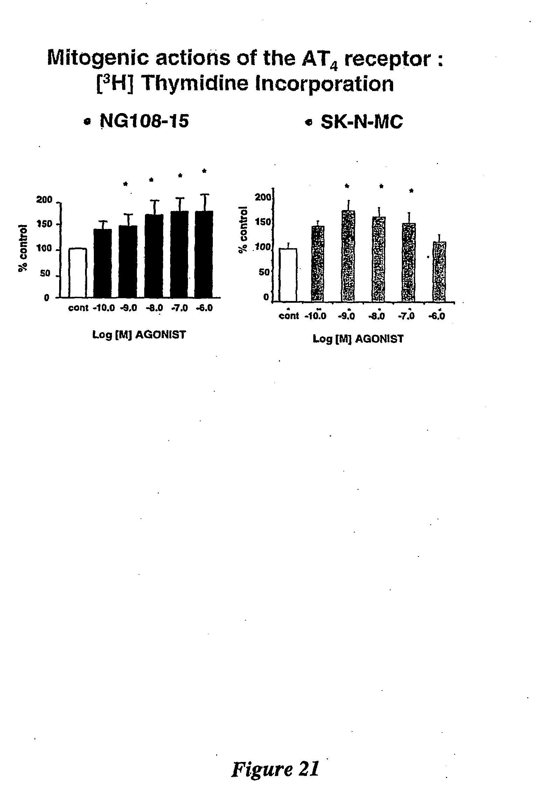

Figure 21 illustrates stimulation of [³H]-thymidine incorporation in NG108-15 or SK-N-MC cells following exposure to 10⁻¹⁰ to 10⁻⁶ M LVVYPWTQRF (SEQ ID NO:2). Values are the mean ± SEM of several experiments performed in quadruplicate, and are expressed as a percentage of control. *p<0.05.

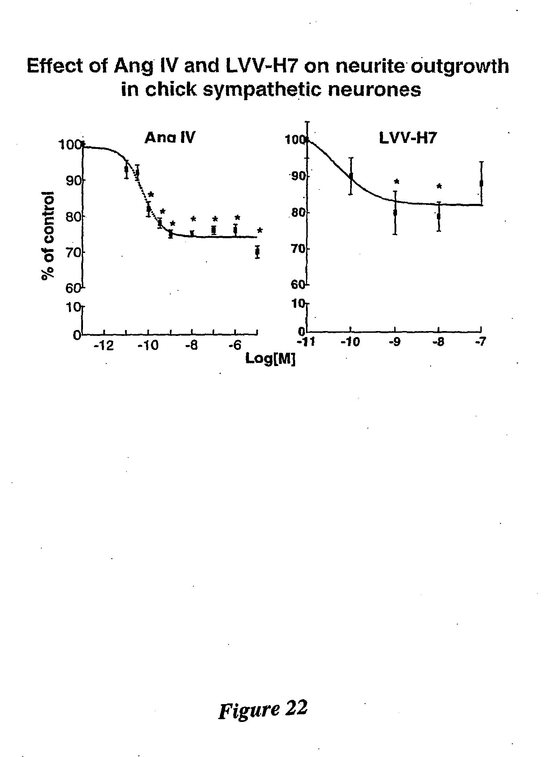

Figure 22 summarises the effect of LVVYPWTQRF (SEQ ID NO:2) and Ang IV on neurite outgrowth from E15 chick sympathetic neurones. The values are expressed as % of control.

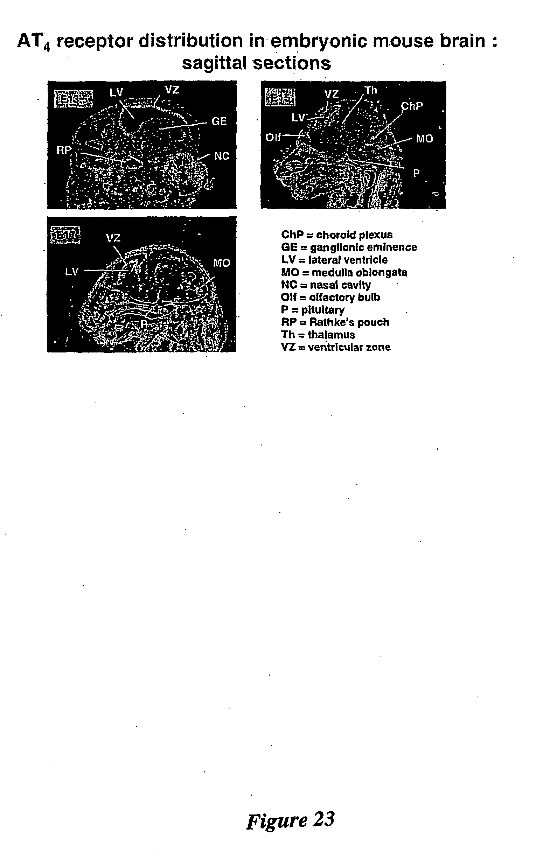

Figure 23 shows the distribution of AT₄ receptor/IRAP in the developing mouse brain, from embryonic day 13.5 to 17. The sections were cut in the sagittal plane.

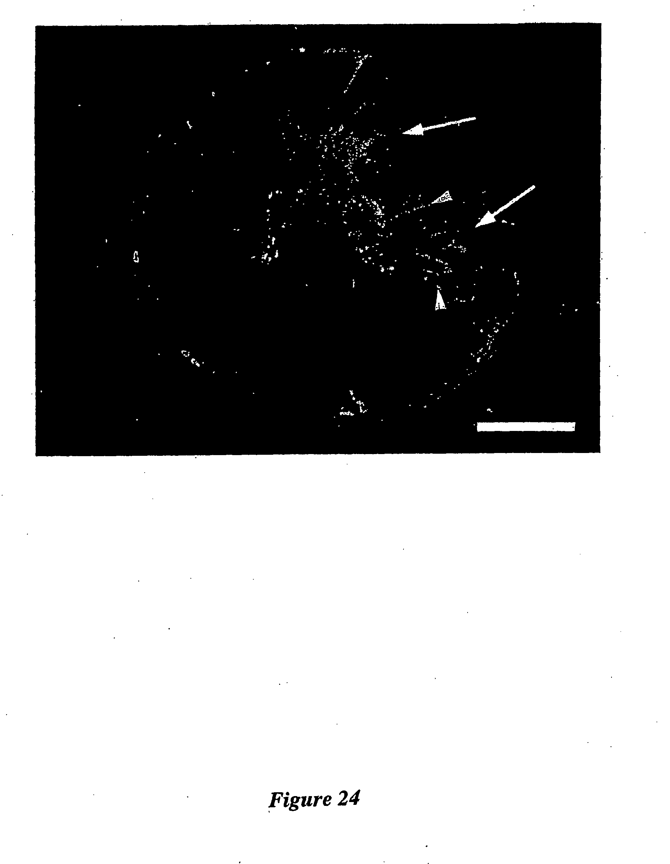

Figure 24 illustrates [¹²⁵I]-Ang IV binding to a section of damaged sheep spinal cord. The arrowheads indicate binding to blood vessels, and the arrows indicate activated glia. Scale bar = 1.9 mm.

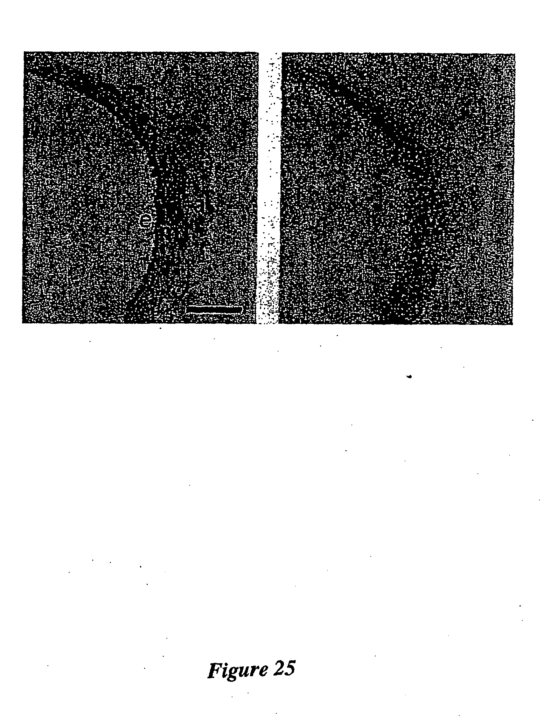

Figure 25 illustrates [¹²⁵I]-Ang IV binding to an uninjured rabbit carotid artery. The left figure is a haematoxylin and eosin-stained section. The right figure represents a haematoxylin and eosin-stained section which has been overlaid with the corresponding X-ray film exposed to the incubated section. e=endothelial cells, m=media, a=adventitia. Scale bar=0.15mm.

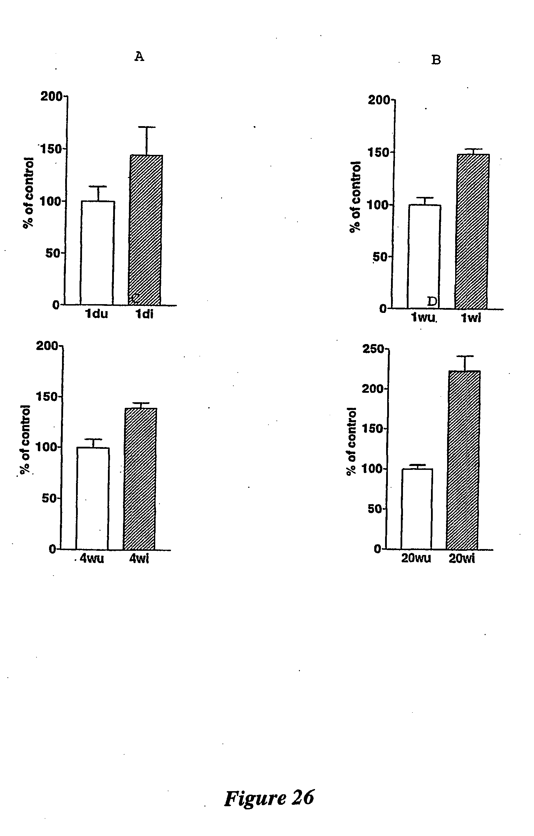

Figure 26 represents the results of quantification of [¹²⁵I]- Ang IV binding in the media/ neointima of the right carotid artery of rabbits, (A) 1 day (1di), (B) 1 week (1wi), (C) 4 weeks (4wi) and (D) 20 weeks (20wi) after balloon injury. Values are the mean ± SEM of 3-4 animals, and are expressed as a percentage of control tissues (the left carotid arteries): 1 day untreated (1du), 1 week untreated (1wu), 4 weeks untreated (4wu) and 20 weeks untreated (20wu) * p<0.05.

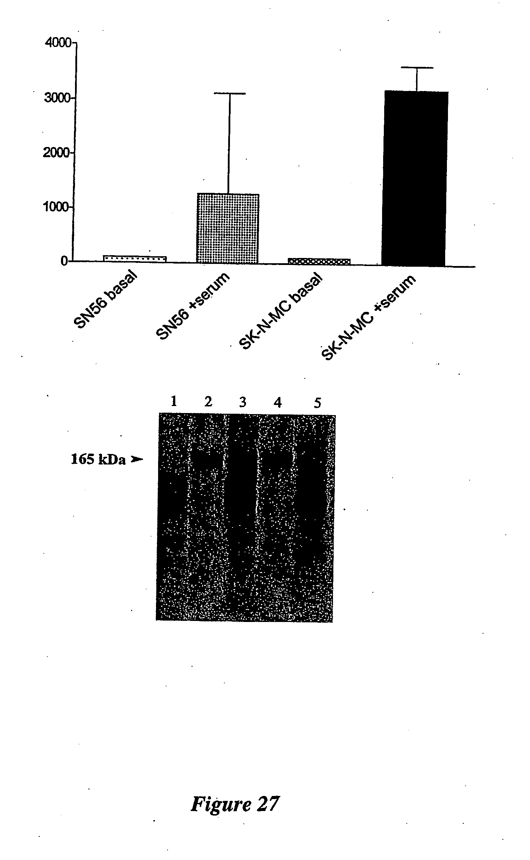

Figure 27 shows the stimulation of cell surface expression of IRAP by serum in SN56 and SK-N-MC cell lines detected by an ELISA assay. Results are expressed as percent of basal (Top panel). Presence of IRAP (arrowhead) in high density microsomes (2), low density microsomes (3) and plasma membrane (4) in SK-N-MC cells after serum stimulation detected by Western blot analysis. Note its absence in cytoplasm (1) and nucleus and mitochondria (5) (bottom panel).

### DETAILED DESCRIPTION OF THE INVENTION

The invention will now be described in detail by way of reference only to the following non-limiting examples and drawings.

We have purified the AT₄ receptor by anion exchange and wheat germ lectin affinity chromatography, and determined its amino acid sequence by tandem mass spectrometry of tryptic peptides. A search of the EMBL protein sequence database found that 18 of the 19 amino acid residues comprising the derived sequence were identical to residues 978-996 of human oxytocinase TrEMBL accession # 000769), which is the homologue of insulin-regulated aminopeptidase (IRAP).

To confirm that IRAP is indeed the AT₄ receptor, we have transfected expression plasmids containing the IRAP cDNA into HEK 293 cells and Chinese hamster ovary (CHO) cells, and found a 10-20 fold increase in sites which specifically bind ¹²⁵I-Nle¹ Ang IV. The binding of ¹²⁵I-Nle¹ angiotensin IV to these sites can be competitively inhibited by ligands of AT₄ receptors, including Ang IV, LVVYPWTQRF (SEQ ID NO:2) and KYLPGPLQ (SEQ ID NO:3). IRAP binds Ang IV with an affinity of 20 to 30 nM. We have demonstrated by cross-linking studies that the AT₄ receptor, like IRAP, is a glycoprotein of molecular size 165 kDa. We were able to cross-link ¹²⁵I-Nle Ang IV-BP to HEK 293-T cells which had been transfected with IRAP cDNA in an expression plasmid, but not to cells transfected with control plasmids.

We have also demonstrated, using *in situ* hybridization histochemistry with antisense oligonucleotides and by immunohistochemistry using an antibody raised against mouse IRAP, that the distribution of IRAP and its mRNA parallel that of the AT₄ receptor. We have therefore demonstrated that IRAP is the AT₄ receptor.

We have also shown that Nle-Ang IV and LVVYPWTQRF (SEQ ID NO:2) inhibit the aminopeptidase activity of IRAP, *in vitro*. We therefore believe that *in vivo* modulation of the enzyme by peptides such as Ang IV, Nle¹-Ang IV, LVVYPWTQRF (SEQ ID NO: 2) and KYLPGPLQ (SEQ ID NO:3) activates neurones in the central nervous system to enhance learning and memory. Structure-activity studies performed on these peptides and on known inhibitors of leucine-aminopeptidases will enable us to develop peptidomimetic and other drugs which modify the IRAP enzymatic activity.

This invention unites findings on the effects of peptide and non-peptide ligands of the AT₄ receptor on the central nervous, cardiovascular and renal systems with the effects of
(a) candidate substrates of IRAP, and
(b) protein constituents of GLUT4 vesicles.

Both IRAP and GLUT4 are known to translocate to the cell surface in response to insulin in adipocytes, skeletal muscles and cardiac myocytes, which is the predominant glucose-transporting mechanism in these cells. Although the roles of IRAP in these cells are unknown, its close association with GLUT4 is suggestive of a role for the enzyme in the regulation of glucose uptake and its storage.

Numerous conclusions can be drawn from our results. For example, the dramatic effects of AT₄ receptor ligands on memory and learning in normal animals and reversal of amnesia induced by cholinergic antagonists and bilateral perforant pathway lesions may be mediated by
(1) modulation of glucose transport in neurones
(2) modulation of extracellular and intracellular concentrations of neuropeptides such as vasopressin, oxytocin, somatostatin, angiotensin III, Lys-bradykinin, neurotensin, met-enkephalin, dynorphin A, neurokinin A, neuromedin B, and cholecystokinin 8, which are all thought to be substrates of the enzyme *in vitro,* and/or
(3) modulation of the levels of polypeptides such as insulin, IGF-I and IGF-II.

The activity of the AT₄ receptor may also be regulated by proteins known to interact with IRAP, such as tankyrase; proteins which are associated with GLUT4 vesicles, such as sortilin and IGF-II receptor; and proteins which regulate GLUT4 vesicle translocation, such as Munc 18c, Syntaxin 4, SNAP23 , Synip, and VAMP 2 (Pessin et al, 1999, Cheatham, 2000, Foster and Klip, 2000, Simpson et al, 2001, Holman and Sandoval, 2001).

This invention relates to novel methods for the development of modulators of AT₄ receptor/IRAP activity as claimed in claim 1. These compounds may lead to an alteration in the levels of
(1) a number of neuropeptides, such as arginine-vasopressin, oxytocin, somatostatin, angiotensin III, Lys-bradykinin, neurotensin, met-enkephalin, dynorphin A, neurokinin A, neuromedin B, cholecystokinin 8, and
(2) a number of polypeptides, such as insulin, insulin-like growth factor-I and insulin-like growth factor-II.

### The development of modulators of AT₄

receptor/IRAP activity may for example be based on
(a) modification of peptides which bind to AT₄ receptor/IRAP, such as VYIHPF (SEQ ID NO:1), LVVYPWTQRF (SEQ ID NO:2), and KYLPGPLQ (SEQ ID NO:3);
(b) peptidomimetic compounds based on the peptide structures of known modulators of IRAP;
(c) compounds based on the structure of known inhibitors of the family of leucine aminopeptidases, such as L-leucinethiol, L-leucinal, or L-Ieucinol; and
(d) compounds which interfere with the interaction of IRAP with other proteins which regulate IRAP activity

The invention provides an assay to screen for compounds which modulate the activity of IRAP as amended in claim 1. Also disclosed is a diagnostic marker for disorders related to alterations in the activity of IRAP in serum or in tissues.

The modulators of IRAP activity preferably result in a decrease in the catalytic activity of IRAP. These compounds are useful for increasing serum and tissue levels of biologically active agents such as arginine-vasopressin, oxytocin, somatostatin, angiotensin III, Lys-bradykinin, neurotensin, met-enkephalin, dynorphin A, neurokinin A, neuromedin B, cholecystokinin 8, insulin, IGF-I or IGF-II. Therefore administration of modulators of IRAP activity can have the same effects or uses as administration of exogenous arginine-vasopressin, oxytocin, insulin, IGF-I or IGF-II.

Compounds which modulate the activity of IRAP may be used in the treatment, prevention or diagnosis of conditions such as
1. neurodegenerative disorders, including Alzheimer's disease and other forms of dementia;
2. other disorders of the CNS, particularly those involving motor and sensory systems;
3. disorders of the CNS resulting from trauma or stroke;
4. disorders of the cardiovascular system, including cardiac hypertrophy, congestive heart failure, hypertension, and atherosclerosis;
5. disorders of development and/or growth;
6. disorders of the reproductive tract;
7. disorders associated with pregnancy;
8. disorders of glucose and fat metabolism, including syndrome X, hyperlipidemia, and diabetes;
9. cancer; or
10. premature or delayed labour.

Such compounds may also be used to accelerate wound healing. Unless otherwise indicated, the following examples use conventional molecular biology, cellular biology, and recombinant DNA techniques. Such techniques are well known to the skilled worker, and are explained fully in the literature. See, for example , Maniatis, Fritsch & Sambrook, "Molecular Cloning: A Laboratory Manual" (1982); "DNA Cloning: A Practical Approach," Volumes I and II (D.N. Glover, ed., 1985); "Oligonucleotide Synthesis" (M.J. Gait, ed., 1984); "Nucleic Acid Hybridisation" (B.D. Hames & S.J. Higgins, eds., 1985); "Transcription and Translation" (B.D. Hames & S.J. Higgins, eds., 1984); "Animal Cell Culture" (R.I. Freshney, ed., 1986); "Immobilised Cells and Enzymes" (IRL Press, 1986); B. Perbal, "A Practical Guide to Molecular Cloning" (1984), and Sambrook, et al., "Molecular Cloning: a Laboratory Manual" (1989). Ausubel, F. et al., 1989-1999, "Current Protocols in molecular Biology" (Green Publishing, New York).

In describing the present invention, the following terminology is used, in accordance with definitions set out below.

The terms "AT₄ receptor", "AT₄ receptor/IRAP" and "IRAP" are used interchangeably. While hitherto they have been regarded as being separate molecules, we have now determined that they are one and the same.

The terms "nucleic acid" and "polynucleic acid" refer herein to deoxyribonucleic acid and ribonucleic acid in all their forms, i.e., single and double-stranded DNA, cDNA, mRNA, and the like.

The terms "antisense" oligonucleotides generally refers to small, synthetic oligonucleotides resembling single-stranded DNA, which are applied to the inhibition of gene expression by inhibition of a target messenger RNA (mRNA). See Milligan, J.F. et al., J.Med.Chem 36 (14), 1923 - 1937 (1993), the relevant portion of which is hereby incorporated by reference. These oligonucleotides inhibit gene expression of target genes. This is generally attained by hybridisation of the antisense oligonucleotides to coding (sense) of a targeted mRNA, as is known in the art. The exogenously administered antisense agents disclosed herein decrease the levels of mRNA and protein encoded by the target gene, and/or cause changes in the growth characteristics or shapes of thus treated cells. See Milligan et al., (1993); Helene, C. and Toulme, J. Biochim. Biophys, Acta 1049, 99-125 (1990); Cohen, J. S. D. Ed., Oligonucleotides as antisense inhibitors of gene expression; CRC Press: Boca Raton, F L (1987), the relevant portions of which are hereby incorporated by reference. As used herein, "antisense oligonucleotide" is generally a short sequence of synthetic nucleotide which hybridises to any segment of a mRNA encoding a targeted protein under appropriate hybridisation conditions, and which upon hybridisation causes a decrease in expression of the targeted protein.

As used herein, the term "treat" or "treating" refers to a treatment which decreases or abolishes the likelihood that a subject administered such treatment will manifest symptoms of an AT₄ receptor/TRAP-related disease.

As used herein, "functionally active" and "functional activity" in reference to the peptides disclosed herein means that the compounds generated therefore are able to modulate the activity of insulin-regulated aminopeptidase (IRAP) The activities of IRAP include
(a) its aminopeptidase activity
(b) AT₄ receptor activity
(c) signalling activity such as activation of second messenger pathways, and
(d) modulating glucose transport. Therefore, a functionally active compound as described herein is capable of competitively inhibiting, stimulating or modulating one or more of these activities.

Amino acid sequence variants of the peptides disclosed herein are prepared by introducing appropriate amino changes into amino acid sequence, or by *in vitro* synthesis. Such variants include, for example, deletions from, or insertions or substitutions of, amino acid residues within the peptide sequences disclosed. Any combination of deletion, insertion, and substitution may be made to arrive at an amino acid sequence variant of the peptides disclosed herein, provided that such a variant possesses the desired characteristics described herein.

Covalent modifications of the peptides disclosed herein are also included. For example, covalent modifications may be intrpduced into the peptides by reacting targeted amino acid residues with an organic derivatizing agent which is capable of reacting with selected amino acid side chains or the N- or C-terminal residues.

Cysteiny1 residues most commonly are reacted with α-haloacetates and corresponding amines, such as chloroacetic acid or chloroacetamide, to give carboxymethyl or carboxyantidomethyl derivatives. Cysteinyl residues also are derivatized by reaction with bromotrifluoroacetone, α-bromo-β-(5-imidozoyl)propionic acid, chloroacetyl phosphate N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are derivatized by reaction with diethyl-pyro-carbonate at pH 5.5-7.0 because this agent is relatively specific for the histidyl side chain. Para-bromophenacyl bromide also is useful; the reaction is preferably performed in 0.1M sodium cacodylate at pH 6.0. Lysinyl and amino terminal residues are reacted with succinic or other carboxylic acid anhydrides. Derivatization with these agents has the effect of reversing the charge of the lysinyl residues. Other suitable reagents for derivatizing α-amino-containing residues include imidoesters such as methyl picolinimidate; pyridoxal phosphate; pyridoxal; chloroborohydride; trinitrobenzenesulfonic acid; O-methylisourea; 2,4-pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine epsilon-amino group.

The specific modification of tyrosyl residues may be made, with particular interest in introducing spectral labels into tyrosyl residues by reaction with aromatic diazonium compounds or tetranitromethane. Most commonly, N-acetylimidizole and tetranitromethane are used to form O-acetyl tyrosyl species and 3-nitro derivatives, respectively.

Carboxyl side groups such as aspartyl or glutamyl are selectively modified by reaction with carbodiimides (R'-N=C=N-R'), where R and R' are different alkyl groups, such as 1-cyclohexyl-3-(2-morpholinyl-4-ethyl) carbodiimide or 2-ethyl-3-(4-azonia-4,4-dimethylpentyl) carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues respectively. Alternatively, these residues are deamidated under mildly acidic conditions. Either form of these residues falls within the scope of this invention.

Other modifications include hydroxylation of proline and lysine, phosphorylation of hydroxyl groups of seryl or threonyl residues, methylation of the α-amino groups of lysine, arginine, and histidine side chains, acetylation of the N-terminal amine, and amidation of any C-terminal carboxyl group. See Creighton, Proteins: Structure and Molecular Properties, pp.79-86 (W.H. Freeman & Co., 1983).

The term "more resistant to metabolic degradation" means that the compound has been modified such that the resulting compound is more stable under acidic conditions than the "native" sequence of the peptides. For example, amino acid substitutions as discussed previously may be undertaken which produce compounds more resistant to metabolic degradation. It is well known in the art that D-amino acids, and amino acid analogues are more resistant to acidic environments. Conjugates of small peptides and cholic acid have reduced metabolic degradation problems.

The term "biological activity" with reference to AT₄ receptor/IRAP means a biological activity which is normally promoted, either directly or indirectly, by the presence of AT₄ receptor/IRAP, and includes, but is not limited to, AT₄ receptor/IRAP binding and biochemical effects such as modulation of the enzymatic, signalling, and glucose transport activities of IRAP, and modulation of the AT4 receptor activities, including enhancement of memory, reversal of amnesia and growth promotion. Such biological activities can be measured by conventional *in vitro* and *in vivo* assays, including enzymatic assays using a synthetic substrate, peptide degradation assays using HPLC, behavioural assays for memory and learning such as the Barnes maze, and glucose uptake assays in cells, synaptosomes and whole animals.

It will be cleanly understood that the compounds disclosed may be synthesised by any suitable method. Solid phase methods, such as those developed for synthesis of peptides and peptidomimetic compound, are preferred, including but not limited to the Fmoc solid phase peptide synthesis method described herein, the Boc solid phase peptide synthesis method, and pin synthesis methods (for review, see Maeji *et al*., 1995) . Those skilled in the art will readily be able to select the most suitable method for any given compound.

In one example, the phosphodiester residues of the antisense oligonucleotide are modified or substituted. Chemical analogues of oligonucleotides with modified or substituted phosphodiester residues, eg, to the methylphosphonate, which increase the *in vivo* stability of oligonucleotide are particularly preferred. The naturally occurring phosphodiester linkages of oligonucleotides are susceptible to some degree of degradation by cellular nucleases. Many of the residues proposed herein, on the contrary, are highly resistant to nuclease degradation. See Milligan et al, and Cohen, J. S D., Supra. In another example the oligonucleotides may be protected from degradation by adding a "3'-end cap", by which nuclease-resistant linkages are substituted for phosphodiester linkages at the 3' end of the oligonucleotide See Tidd D. M. and Warenrus, H. M., Be. J. Cancer 60:343-350 (1989); Shaw, J.P. et al., Nucleic acids research. 19:747-750 (1991),

Phosphoroamidates, phosphorothioates, and methylphosphonate linkages all function adequately in this manner. The more extensive the modification of the phosphodiester backbone, the more stable the resulting agent, and in many instances the higher their RNA affinity and cellular permeation. See, Milligan et al., supra. Thus the number of residues which may be modified or substituted will vary, depending on the need, target and route of administration, and may be from one to all residues, or any number in between. Many different methods for replacing the entire phosphodiester backbone with novel linkages are known. See Milligan et al., Supra. Preferred backbone analogue residues include phosphorothioate, formacetal boranophosphate, 3'-thioformacetal, 5'-thioether, carbonate, 5'-N-carbamate, sulphate, sulfonate, sulfamate, sulfonamide, sulfone, sulfite, 2'-O methyl, sulfoxide, sulfide, hydroxylamine, methylene (methylimino) (MMI), methyleneoxy (methylimino) (MOMI) residues. Phosphorothioate and methylphosphate-modified oligonucleotides are particularly preferred, due to their availability through automated oligonucleotide synthesis. See Milligan et al., Supra. Where appropriate the agents disclosed herein may be administered in the form of their pharmaceutically acceptable salts, or as a mixture of the antisense oligonucleotide and its salt. In a particular example, a mixture of different antisense oligonucleotides or their pharmaceutically acceptable salts is administered.

The antisense nucleic acid molecules (agents) disclosed herein may be of any suitable length, for example about 7 to about 60 nucleotides long, preferably about 12 to about 45, more preferably up to about 30 nucleotides long, and still more preferably up to about 21, although they may be other lengths as well, depending on the particular target and the mode of delivery. The agents disclosed herein may be directed to any and all segments of a target RNA.

In a further example, the oligonucleotide is operatively linked to an agent or molecule which is itself internalised or taken up by living cells. In this manner, the uptake of the agent is enhanced. Examples of agents or molecules suitable for use with the oligonucleotides disclosed herein are transferrin asialoglycoprotein and streptavidin. Other agents will be known to those skilled in the art.

The inventors also disclose a pharmaceutical composition comprising an antisense oligonucleotide in an amount effective to reduce expression of a target mRNA, by passing through a cell membrane and binding specifically with target mRNA in the cells, so as to prevent its translation, together with a suitable pharmaceutically acceptable carrier, eg. sterile pyrogen-free saline solution. The agent may be formulated with a hydrophobic carrier capable of passing through a cell membrane, eg in a liposome, with the liposomes carried in a pharmaceutically acceptable aqueous carrier. The oligonucleotides may be coupled to an agent which inactivates mRNA, such as a ribozyme. Such oligonucleotides may be administered to a subject in need of such treatment to inhibit the activation of specific receptors, enzymes and/or proteins and/or factors. The pharmaceutical formulation may also comprise chimeric molecules comprising antisense oligonucleotides attached to molecules which are known to be internalised by cells. These oligonucleotide conjugates utilise cellular uptake pathways to increase intercellular concentrations of the oligonucleotide. Examples of molecules used in this manner include macromolecules, including transferrin, asialoglycoprotein (bound to oligonucleotides via polylysine) and streptavidin. The antisense compound may be contained in the pharmaceutical formulation within a lipid particle or vesical, such as a liposome or microcrystal. The particles may be of any suitable structure, such as unilamellar or plurilamellar. Preferably, the antisense oligonucleotides is comprised within the liposome. Positively charged lipids such as N-[1-(2,3-dioleoyloxi)propyl]-N,N,N-trimethylammoniumethylsulfate, or "DOTAP", are particularly preferred for such particles and vesicles. However, others are also suitable. The preparation of such lipid particles is well-known. See, for example, US Patent Nos. 4,880,635 to Janoff et al, 4,906,477 to Kurono et al., 4,911,928 to Wallach, 4,917,951 to Wallach, 4,920,016 to Allen et al., 4,921,757 to Wheatley et al., the relevant sections.

The pharmaceutical compositions disclosed herein may comprises the antisense oligonucleotides described above and one or more surfactants. Suitable surfactant components for enhancing the up-take of the antisense oligonucleotides disclosed herein include synthetic and natural as well as full length and truncated forms of surfactant protein A, surfactant protein B, surfactant protein C, surfactant protein D and surfactant protein E, di-saturated phosphatidylcholine (other than dipalmitoyl, dipalmitoyl phatidylcholine, phosphatidylcholine, phosphatidylglycerol, phosphatidylinositol, phosphatidylethanolamine, phosphatidylserine; phosphatidic acid, ubiquinones, lysophoaphatidylethanolamine, lysophosphatidylcholine, palmitoyllysophosphatidylcholine, dehydroepiandrosterone, dolichols, sulfatidic acid, glycerol-3-phosphate, dihydroxyacetone phosphate, glycerol, glycero-3-phosphocholine, dihydroxyacetone, palmitate, cytidine diphosphate (CDP) diacylglycerol, CDP-choline, choline, choline phosphate; as well as natural and artificial lamelar bodies which are the natural carrier vehicles for the components of surfactant, omega-3 fatty acids, polyenic acid, polyenoic acid, lecithin, palmitinic acid, non-ionic block copolymers of ethylene or propylene oxides, polyoxypropylene, monomeric and polymeric, polyoxyethylene, monomeric and polymeric, poly (vinyl amine) with dextran and/or alkanoyl side chains, Brij 35, Triton X-100 and synthetic surfactants ALEC, Exosurf, Survan and Atovaquone, among others. These surfactants may be used either alone or as part of a multiple component surfactant in a formulation, or as covalently bound additions to the 5' and/or 3' ends of the antisense oligonucleotides (oligos).

As already indicated, the agent disclosed herein may also provided as a composition, comprising the agent and a carrier. The carrier is preferably a biologically acceptable carrier, and more preferably a pharmaceutically or veterinarily acceptable carrier in the form of gaseous, liquid, or solid carriers, and mixtures thereof, which are suitable for the different routes of administration intended. The composition may optionally comprise other agents such as other therapeutic compounds known in the art for treatment of the condition or disease, antioxidants, flavouring and colouring agents, fillers, volatile oils, buffering agents, dispersants, surfactants, RNA-inactivating agents, antioxidants, flavouring agents, propellants and preservatives, as well as other agents known to be utilised in therapeutic compositions. An example of the mRNA inactivating agent is an enzyme, such as ribozyme.

The composition generally contains the antisense oligonucleotide in an amount of about 0.01 to about 99.99 w/w, preferably about 1 to about 40 w/w, and more preferably about 5 to about 20 w/w of the composition. However, other ingredients, and other amounts of the agent are also suitable within the confines of this invention.

The agent may be provides in various formulations which are tailored for different methods of administration and routes of delivery. The formulations contemplated are, for example, a transdermal formulation also containing carrier(s) and other agents suitable for delivery through the skin, mouth, nose, vagina, anus, eyes, ears, other body cavities, intradermally, as a sustained release formulation, intracranial, intrathecally, intravascularly, by inhalation, intrapulmonarily, into an organ, by implantation, including suppositories, creams, gels, and the like, as is known in the art. In one particular formulation, the agent may be suspended or dissolved in a solvent. In another the carrier may comprises a hydrophobic carrier, such as lipid particles or vesicles, including liposomes and micro crystals. The preparation of all of these formulations, as well as the ingredients to be utilised, are known in the art, and need not be further described here. In one particularly preferred example of the vesicle formulation, the vesicles comprise liposomes containing the antisense oligonucleotdies. The lipid vesicles may comprise N-(1-[2,3-dioleoxyloxi]propyl)-N,N,N-trimethyl-ammonium methylsulfate as well as other lipids known in the art to provide suitable delivery of DNA to target cells. In one example, the formulation comprises a respirable formulation, such as an aerosol. The agent, composition, and formulation disclosed herein may be provided in bulk, and in unit form, as well as in the form of an implant, a solution or suspension, a capsule or cartridge, which may be openable or piercable as is known in the art. A kit is also disclosed, which comprises a delivery device, and in separate containers, the agent, composition or formulation , and optionally other agents, and instructions for the use of the kit components.

The kit may optionally also comprise in a separate container an agent selected from the group consisting of other therapeutic compounds, antioxidants, flavouring and colouring agents, fillers, volatile oils, buffering agents, dispersants, surfactants, cell internalisation or up-take agents, RNA-inactivating agents, antioxidants, flavouring agents, propellants and preservatives, among other suitable additives for the different formulations. When a solvent for the agent or other ingredients is added, organic solvents and organic solvents mixed with one or more co-solvents may be utilised as well as aqueous solvents as is known in the art. The agent disclosed herein may be provided in conjunction with a vector for delivery purposes, or for manufacturing copies thereof. The agent may be operatively linked to the vector, using methods known in the art. The agent may also be provided within a host cell for amplification of the oligonucleotide, and for storage purposes.

### EXAMPLE 1 PURIFICATION AND IDENTIFICATION OF THE LIGAND-BINDING SUBUNIT OF THE AT₄ RECEPTOR AND INTERACTING PROTEINS

Since the DNA sequences encoding the AT₄ receptor had not been previously identified, we wished to purify the receptor protein and obtain peptide sequence by mass spectrometry, which would allow us to identify the gene encoding the receptor. The purification procedure is described below.

Bovine adrenals were homogenised with a polytron homogeniser in hypotonic buffer (50mM Tris-HCl pH 7.4, 5mM EDTA, pH 7.4). Tissue debris was removed by centrifugation at 600g for 5min at 4°C. The supernatant was centrifuged at 50,000g for 20min at 20°C and the membrane pellet was resuspended in isotonic buffer consisting of 50mM Tris-HCl pH 7.4, 150mM NaCl, 5mM EDTA, 20µM bestatin, 100µM PMSF, 0.1% BSA.

To covalently cross-link [¹²⁵I]-Nle¹-BzPhe⁶-Gly⁷-Ang IV-benzoylphenylalanine ([¹²⁵I]-Nle-Ang-IV-BP) to the receptor, bovine adrenal membranes (1.6µg of protein) were incubated with 0.1µCi [¹²⁵I] -Nle-Ang-IV-BP in 20ml of isotonic buffer in Eppendorf tubes (1ml per tube) at 37°C with shaking for 90min in the dark. Membranes were collected by centrifugation at 60,000g for 10min at 4°C, and resuspended in 500µl of ice cold isotonic buffer and UV irradiated at 365nm on ice for 2h. Membranes were again collected by centrifugation at 60,000g for 20 min at 4°C.

To solubilise the receptor, cross-linked membranes were homogenised in 2ml solubilisation buffer (1% CHAPS, 20mM Tris-HCl, pH 7.5, 5mM EDTA) in a Dounce homogeniser, and shaken at room temperature for 48h. Insoluble material was pelleted by centrifugation at 100,000g for 1h at 4°C. Non-crosslinked membranes (48mg of protein) were solubilised and centrifuged similarly, and the supernatant combined with that from cross-linked membranes.

To enrich for the receptor, solubilised membrane protein was applied to a 1ml DEAE fast flow anion exchange column. The column was then washed with 5ml of solubilisation buffer, then with 5ml of solubilisation buffer plus 50mM NaCl. AT₄ receptor was eluted with solubilisation buffer plus 150mM NaCl and collected in 0.5ml fractions. Proteins in 200µl aliquots of eluted fractions were resolved by 7.5% SDS-PAGE and the gel stained first with Coomassie brilliant blue and then overstained with silver nitrate (Shevchenko et al. 1996). Radioactive bands were imaged after exposing to phophorimager plates for 16h, as illustrated in Figure 1.

The silver-stained band that co-migrated with the major radioactive band at 165 kDa was analysed by mass spectrometry as follows.

The protein band was excised, reduced, alkylated and digested with trypsin *in situ* (Moritz *et al.*, 1996). Tryptic peptides were subjected to capillary-column reversed phase-high performance liquid chromatography (RP-HPLC; Hewlett Packard Model 1090A modified for capillary chromatography), coupled on-line with an electrospray ionization (ESI) ion trap (IT) mass spectrometer (LCQ Finnigan MAT, San Jose, CA, USA) for peptide sequencing. The column used was a 150 x 0.20mm ID capillary column (Brownlee RP-300, 7µm C8) manufactured using a PVDF end frit. A 60 min linear gradient (flow rate 1.7µL/min) was used from 0-100% B, where solvent A was 0.1% v/v aqueous TFA and solvent B was 0.1% v/v aqueous TFA in 60% acetonitrile. The ESI parameters were as follows: spray voltage, 4.5kV; sheath gas and auxiliary gas flow rates, 5 and 30 (arbitrary value), respectively; capillary temperature, 150°C; capillary voltage, 20V; and tube lens offset 16V. The sheath liquid used was 2-methoxyethanol (99.9% HPLC grade) delivered at a flow rate of 3µL/min. The electron multiplier was set to -860 V. In both MS and MS/MS modes, the trap was allowed a maximum injection time of up to 200ms. The automatic gain control parameter was turned on for all experiments, ensuring that the number of ions in the trap was automatically kept to a constant preset value. The range scanned in MS mode was 350-2000 Da, and in MS/MS the range varied according to the mass of the ion selected for MS/MS. After acquiring one scan in MS, the most intense ion in that spectrum above a threshold of 1x10⁵ was isolated for subsequent zoom scan to determine charge state, then collision-induced dissociation or MS/MS in the following scan. The dissociation energy for MS/MS was set to 55%. All spectra were recorded in centroid mode.

Two MS/MS spectra were identified using the SEQUEST algorithm (Eng et al, 1994; incorporated into the Finnigan Xcalibur-Biomass software) and the Ludwig Institute non-redundant database (Ludwig Institute for Cancer Research, Lausanne branch). These peptides were identified as residues 208-220 and 321-349 of CD34 from *Bos taurus* (TrEMBL accession number Q9XS61), as illustrated in Figure 2. One MS/MS spectrum not identified by SEQUEST was deduced by manual amino acid sequence interpretation to be AHLSEVQAFFENQSEATFR (SEQ ID NO:5). A search of the database revealed 18 of the 19 amino acids to be identical to residues 979-996 of human oxytocinase (TrEMBL accession number 000769), the human homologue of rat insulin-regulated aminopeptidase (IRAP) (Figure 2). The derived bovine sequence has an alanine residue at the N-terminus, whereas the human sequence has a threonine.

Further enrichment of the AT₄ receptor was achieved by coupling anion exchange chromatography to wheat germ lectin affinity chromatography, as described in Example 3.

### EXAMPLE 2 CONFIRMATION THAT THE AT₄ RECEPTOR IS IRAP

To confirm that the AT₄ receptor is IRAP, we obtained plasmids containing the human IRAP cDNA (Rogi et al, 1996) from Dr Masafumi Tsujimoto, and transfected them into HEK 293 cells. Membranes prepared from these cells were incubated with ¹²⁵I-Nle Ang IV in the absence and presence of increasing concentrations of Ang IV (Figure 3) LVVYPWTQRF (SEQ ID NO:2) (Figure 4) and KYLPGPLQ (SEQ ID NO:3) (Figure 5) respectively. The amount of radioligand bound to the membranes from HEK 293 cells transfected with IRAP cDNA was expressed as a percentage of radioligand bound to membranes from HEK 293 cells transfected with control plasmids. Cross-linking experiments were also carried out on the membranes from HEK 293 cells transfected with the IRAP cDNA, using the method described in Example 1 (Figure 6).

Membranes from HEK 293 cells transfected with IRAP cDNA produced a 25-35 fold increase in the total number of binding sites, compared to membranes prepared from cells transfected with control plasmids. The affinities of Ang IV and LVVYPWTQRF (SEQ ID NO:2) in competing for the binding of ¹²⁵I-Nle Ang IV in the IRAP-transfected cells were not significantly different from that of the endogenously-occurring IRAP in HEK 293 cells (Figures 3 and 4). The cross-linking experiment revealed that at least two radiolabelled bands were present on the SDS-PAGE gel, a major band of approximately 165 kDa and a minor band of greater than 250 kDa (Figure 6). These results demonstrated a dramatic increase in AT₄ receptor in the HEK 293 cells transfected with the IRAP cDNA, and confirmed that IRAP is the AT₄ receptor.

We also investigated the distribution of IRAP, using an antibody raised in rabbits against a GST fusion protein containing the cytoplasmic tail of murine IRAP, and the distribution of IRAP mRNA, using *in situ* hybridization with antisense oligonucleotides generated from the mouse IRAP cDNA sequence. The antisense sequences used include
5'GAGGTTAGGTTTGGATGTAGGCTAAGTTCAT 3' (SEQ ID NO:6) and
5'ATTGATCAGGTTGGCTCGGTCTTTGTCA 3' (SEQ ID NO:7)
from the mouse IRAP cDNA sequence retrieved from the CELERA database. The distribution of IRAP-positive immunoreactivity and IRAP mRNA closely correlated with the distribution of AT₄ receptor, as determined using ¹²⁵I-Nle Ang IV binding in mouse brain (Figure 7) and embryo (Figure 8). The distribution of IRAP mRNA in human cerebellum was demonstrated by *in situ* hybridization histochemistry, using antisense and sense oligonucleotide sequences derived from the human IRAP cDNA sequence. The antisense sequences used include nucleotides 1136-1109-, nucleotides 1407-1377 and nucleotides 2587-2560 of the human IRAP sequence (Human Genbank Acc.U62768). The adjacent sections of the cerebellum were labelled with ¹²⁵I-Nle Ang IV to demonstrate AT₄ receptor binding. The results are shown in Figure 9.

### EXAMPLE 3 DIFFERENT ISOFORMS OF AT₄ RECEPTOR/IRAP

The affinity of AT₄ receptor/IRAP for its ligands varies considerably between tissues and cell types. This may be due to interactions of the ligand binding subunit with other proteins which modulate the binding affinity. Alternatively, different isoforms of the enzyme may exist. In support of the latter conclusion, an IRAP-related EST (Genbank accession #BF381719) which includes intron 2 sequence following exon 1, was identified from a library made from a cell line derived from human primitive neuroectoderm. This could represent a tissue- or stage-specific variant of IRAP arising by alternative splicing. The existence of brain-specific variant of AT₄ receptor/IRAP may make it possible to develop modulators of AT₄ receptor/IRAP which specifically target the brain form of the receptor, and which therefore do not have unwanted side-effects in peripheral tissues.

### EXAMPLE 4 IDENTIFICATION OF PROTEINS WHICH INTERACT WITH THE LIGAND-BINDING SUBUNIT OF IRAP/AT₄ RECEPTOR BY SDS-PAGE GELS AND IDENTIFICATION BY MASS SPECTROMETRY

When AT₄ receptor/IRAP from bovine adrenal membranes was cross-linked to [¹²⁵I]-Nle-Ang IV-BP and solubilised in 1% NP40 or 1% CHAPS and the proteins resolved by SDS-PAGE, one, two or three minor radiolabelled higher molecular weight complexes were observed (Figure 10). These migrated with apparent molecular weights ranging from approximately 180 to 300kDa. These results suggested that the ligand-binding subunit of AT₄ receptor/IRAP existed as a multi-subunit complex. A 250kDa complex was also observed when the AT₄ receptor/IRAP from SK-N-MC cells was cross-linked to [¹²⁵I]-Nle-Ang IV-BP and the proteins resolved by SDS-PAGE under non-reducing conditions, but not under reducing conditions (Figure 11), suggesting that a subunit interacts with the ligand-binding subunit through disulphide bonds.

In order to determine the mechanism by which AT₄ receptor/IRAP influences other physiological events, the identification of proteins which complex with the ligand binding subunit of IRAP/AT₄ is required. We hypothesise that the interactions may modulate the function of AT₄ receptor/IRAP by inhibiting or enhancing its catalytic activity. Alternatively, the binding of peptides to AT₄ receptor/IRAP may activate a second messenger system via proteins which interact with IRAP. AT₄ receptor/IRAP-interacting proteins might also have roles in the intracellular trafficking of AT₄ receptor/IRAP. Molecules which interact with the AT₄ receptor/IRAP represent potential targets for drugs designed to reverse memory deficits in conditions such as Alzheimer's disease and other forms of dementia, or to modulate other physiological actions of the IRAP/AT₄ system. If an interacting molecule shows a more restricted distribution than does AT₄ receptor/IRAP (e.g. limited to brain or certain regions of the brain), it may provide a more suitable target than is IRAP for a drug which specifically modulates the effects of the IRAP/AT₄ system in a particular tissue or cell type.

To achieve further enrichment for the AT₄ receptor/IRAP, with a view to isolating the higher molecular weight complexes, ion exchange purification was scaled up and fractions enriched for the receptor were further purified by wheat germ lectin affinity chromatography. The two-step purification was carried out as follows: [¹²⁵I]-Nle-Ang IV-BP was cross-linked to the AT₄ receptor in bovine adrenal membranes (93mg protein) and solubilised as described in Example 1. Non-crosslinked receptor from bovine adrenal membranes (254mg of protein) was solubilised and combined with cross-linked receptor. The receptor was enriched by DEAE-Sepharose fast flow anion exchange as described above. Fractions enriched for the receptor were then applied to a 1ml wheat-germ lectin-agarose column, the column was washed with 5ml of solubilisation buffer, and the receptor eluted with 0.5M N-acetylglucosamine, 0.5M NaCl in solubilisation buffer, and collected in 0.5ml aliquots. Proteins in 200µl aliquots of eluted fractions were resolved by 7.5% SDS-PAGE, and the gel stained and autoradiographed as described above. The ligand-binding subunit was visualised by Coomassie blue staining, and bands which co-migrated with two of the higher molecular weight complexes were barely visible after silver staining. These bands were excised from the gel and analysed by capillary-column RP-HPLC coupled with ESI-IT-MS/MS, as described above.

The resulting CID spectra of the peptides were compared with those of proteins in the database.

To identify proteins interacting with AT₄ receptor/IRAP in a tissue of interest, AT₄ receptor/IRAP from that tissue was cross-linked to [¹²⁵I]-Nle-Ang IV-BP, solubilised and purified by ion exchange chromatography and wheat germ lectin affinity,chromatography, as described above, and proteins resolved by SDS-PAGE.

Silver or Coomassie blue-stained bands which co-migrated with radiolabelled bands corresponding to higher molecular weight complexes were excised from the gel and analysed by capillary-column RP-HPLC coupled with ESI-IT-MS/MS; as described above.

Two bands corresponding to 250 and 300 kDa were excised from the SDS-PAGE gel, and subjected to alkylation and digestion with trypsin as described above. Tryptic peptides were subjected to capillary-column reversed phase-high performance liquid chromatography, coupled on-line with an electrospray ionization (ESI) ion trap (IT) mass spectrometer for peptide sequencing as described above.

### For the 250 kDA gel band,

1. Twenty-five MS/MS spectra were identified, and the peptides correspond to internal sequences of voltage-dependent calcium channel alpha-2 delta subunit precursor - *Sus scrofa* (TrEMBL accession number 077773).
2. Four MS/MS spectra were identified, and the peptides correspond to internal sequences of integrin beta-1 (fibronectin receptor beta subunit) - *Bos taurus* (SwissProt P53712).
3. Two MS/MS spectra were identified, and the peptides correspond to internal sequences of cytochrome p450 11A1, mitochondrial precursor (cholesterol side-chain cleavage enzyme) - *Bos taurus* (SwissProt P00189)
4. One MS/MS spectrum was identified, and the peptide corresponded to an internal sequence of receptor Dec205 (Ly75) - *Mus musculus* (TrEMBL Q60767)
5. One MS/MS spectrum was identified, and the peptide corresponded to an internal sequence of dopamine beta-monooxygenase precursor - *Bos taurus* (SwissProt P15101).

### For the 300 kDa gel band,

1. Five MS/MS spectra were identified, and the peptides correspond to internal sequences of cation-independent mannose-6-phosphate receptor precursor - *Bos taurus* (SwissProt P08169)
2. Five MS/MS spectra were identified, and the peptides correspond to internal sequences of voltage-dependent calcium channel alpha-2 delta subunit precursor - *Sus scrofa* (TrEMBL accession number 077773).
3. Four MS/MS spectra were identified, and the peptides correspond to internal sequences of cytochrome p450 11A1, mitochondrial precursor (cholesterol side-chain cleavage enzyme) - *Bos taurus* (SwissProt P00189)
4. One MS/MS spectrum was identified, and the peptide corresponded to an internal sequence of dopamine beta-monooxygenase precursor - *Bos taurus* (SwissProt P15101) .

### EXAMPLE 5 ISOLATION AND IDENTIFICATION OF INTERACTING PROTEINS BY CO-IMMUNO-PRECIPITATION OR IMMUNOAFFINITY CHROMATOGRAPHY USING ANTIBODIES AGAINST IRAP/AT₄ RECEPTOR

To identify proteins interacting with AT₄ receptor/IRAP in tissues of interest, membranes are prepared from these tissues and membrane proteins solubilised with NP40 or CHAPS as described in Example 1, and incubated with antibodies against IRAP and protein G-Sepharose beads. As a control, solubilised membranes are incubated with protein G-Sepharose alone. Precipitated proteins are resolved by SDS-PAGE and detected by Coomassie blue or silver staining. Bands appearing in the test but not in the control lanes are excised and digested with trypsin *in situ*, and tryptic peptides analysed by capillary-column RP-HPLC coupled with ESI-IT-MS/MS, as described in example 1. In a similar approach, proteins complexing with IRAP/AT₄ are isolated by immunoaffinity chromatography using IRAP antibody coupled to a chromatography resin, and identified by tandem MS as described above.

Tankyrase, a Golgi-associated MAP kinase substrate has been shown to interact with IRAP in adipocytes (Chi and Lodish, 2000). Other potential IRAP-interacting proteins include GLUT4 and various proteins involved in GLUT4 trafficking including Syntaxin 4, SNAP23, Synip, Munc18c and VAMP2 (Pessin et al, 1999; Cheatham, 2000; Foster and Klip, 2000; Simpson et al, 2001; Holman and Sandoval, 2001). Whether IRAP complexes with these proteins in brain or other tissues or cells of interest is assessed by blotting SDS-PAGE gels containing proteins purified by immunoaffinity chromatography or immunoprecipitation with IRAP antibodies, as described above, and probing with antibodies against the candidate interacting proteins.

### EXAMPLE 6 USE OF THE YEAST TWO-HYBRID SYSTEM TO IDENTIFY FACTORS THAT INTERACT WITH IRAP/ AT₄ RECEPTOR

Yeast cells are transformed with a vector which expresses the IRAP/AT₄ intracellular or extracellular domain (the bait) as a fusion with a DNA-binding domain, such as LexA, and a cDNA library in a yeast expression vector that directs expression of cDNAs fused to a nuclear localisation signal and a transcriptional activation domain. Cells expressing cDNAs which interact with the bait protein are then selected, based on expression of one or more reporter genes, under the control of the LexA operator. Candidates which activate transcription specifically in the presence of the bait-DNA binding domain hybrid are investigated further.

The biological relevance of interactions identified in the yeast two-hybrid system is assessed by examining whether the interacting protein is co-expressed with AT₄ receptor/IRAP in relevant tissues and cell types and by determining whether the interacting protein is co-immunoprecipitated from relevant tissues or cultured cells by antibodies against IRAP/AT₄.

### EXAMPLE 7 IDENTIFICATION OF ENDOGENOUS PEPTIDES WHICH MODULATE THE ACTIVITY OF IRAP/AT₄ RECEPTOR '

We searched for endogenous peptides in the central nervous system which modulated the activity of AT₄ receptor/IRAP. Conventional peptide purification procedures in combination with the AT₄ radioreceptor assay were used to monitor the receptor binding activity of an extract of sheep brain.

The AT₄ radioreceptor assay was used to screen the sheep brain extracts for AT₄ receptor modulatory activity. The membranes were prepared using bovine adrenal glands obtained from the abattoir. The adrenals were diced, homogenized in ice-cold hypotonic buffer (50mM Tris, 5mM EDTA, pH 7.4) and centrifuged for 10min at 500g. The supernatants were heated to 60°C for 15min, and centrifuged for 20min at 40,000g. The resulting pellets were re-homogenized in hypotonic buffer. Binding assay mixtures comprised 56µg of adrenal membranes, 0.14µCi of [¹²⁵I] Ang IV (approximately 260pM), and 10µl of column eluent in a total volume of 270µl in 50mM Tris buffer, pH 7.4, containing 150mM sodium chloride, 5mM EDTA, 100µm phenylmethyl-sulfonyl fluoride, 20µM bestatin, and 0.1% (w/v) bovine serum albumin. The samples were incubated for 2h at 37°C, and the bound ligand was separated on Whatman GF/B glass fibre filters. The relative potency of the fractions in competing for ¹²⁵I-Ang IV binding was determined from a standard curve, in which known amounts of the unlabelled Ang IV were added in concentrations ranging from 10⁻¹⁰ to 10⁻⁶ M.

The same purification procedure was carried out on sheep cerebral cortex which contained blood, and also on blood-free sheep cerebral cortex, in which the sheep brain was perfused with 10 1 of ice-cold phosphate-buffered saline solution. The brain tissues were homogenized in 2M acetic acid (2ml/g tissue), centrifuged and the supernatant decanted. A preliminary purification was carried out on the brain extracts using a preparative C18 column (55-105mm, Waters). The C18 eluant was lyophilized, reconstituted and subjected to a series of chromatographic steps in which the fractions with the highest AT₄ receptor modulatory activity were carried through to the next purification step. In brief, three successive reverse-phase HPLC steps involving columns of varying pore size were used (Deltapak C18, and Novapak C18), followed by anion exchange then cation exchange, with a final purification on a microbore LC C8 column. The purified active peptides were then sequenced on an Applied Biosystems Model 470 A Protein Sequencer with an on-line Nodel 120A PTH Analyzer.

A decapeptide having the sequence LVVYPWTQRF (SEQ ID NO:2) was purified from the sheep cerebral cortex which contained blood. A search of the database revealed that this sequence was present in the internal sequence of (β,δ,γ and ε globin, and is known as LVV-hemorphin 7. This decapeptide may have resulted from the breakdown of haemoglobin. The purification procedure was repeated on brain which had been perfused to remove all traces of blood, and an octapeptide of sequence KYLPGPLQ (SEQ ID NO:3) was purified. This peptide is a fragment of complexin II, a cytosolic protein located in nerve terminals which is associated with the docking/fusion complex which regulates neurotransmitter release.

Synthetic LVVYPWTQRF (SEQ ID NO:2) and KYLPGPLQ (SEQ ID NO:3) displayed comparable affinities to Ang IV in competing for ¹²⁵I-Ang IV binding to bovine adrenal membranes (approx. 1-5nM), and ¹²⁵I-KYLPGPLQ (SEQ ID NO:3) displayed identical binding patterns to ¹²⁵I-Ang IV, whereas ¹²⁵I-LVVYPWTQRF (SEQ ID NO:2) bound to a subset of ¹²⁵I-Ang IV binding sites.

LVVYPWTQRF (SEQ ID NO:2) is released from β-globin into the circulation in uremic patients undergoing haemofiltration, and in patients undergoing open-heart surgery or cardiopulmonary bypass. Significant levels of the decapeptide are also found in ventricular cerebrospinal fluid of patients with intracerebral and subarachnoid bleeding. The half-life of the decapeptide is between 20-30min. This decapeptide may exert a modulatory effect on the activity of AT₄ receptor/IRAPs in the circulatory and central nervous systems.

### EXAMPLE 8 IDENTIFICATION AND DEVELOPMENT OF OLIGONUCLEOTIDE MODULATORS OF IRAP/AT₄ RECEPTOR ACTIVITY

We have shown that the levels of the AT₄ receptor are down-regulated in vivo by the use of anti-sense technology. Anti-sense oligonucleotides to the human sequence of AT₄ receptor are designed to bind to the specific mRNA and target the mRNA for degradation or the prevention of translation. The invention also includes the ability to specifically regulate the different amino - terminus variants of AT₄ receptor which are encoded by splice variants of the gene by targeting the start site of translation for the differentially-spliced mRNAs. We have used an RNA secondary structure prediction algorithm, mfold, (Zucker, 1989) to identify good binding sites for the oligonucleotides. We also carried out RNA walk of the IRAP mRNA to identify regions of the RNA that contained assymmetrical bulges, short stems and included loop structures based on the predicted secondary structure of the RNA. Examples of some of the oligonucleotides identified using these methods are listed below. SEQ ID NOs:8 to 10 are oligonucleotides targeted to the mRNAs encoding both forms of the protein. SEQ ID NOs:12 and 13 are specific for the mRNAs encoding the different forms of the protein.

| | |
|---|---|
| 5'CCAAGTAAGTGCTCATCTTCACACTCTC 3' * | SEQ ID NO.8 |
| 5'GTGTTACTGTCATACAGAAGTGTCTCCTCTC 3' * | SEQ ID NO.9 |
| 5'CAGTTTCATGGCAGTAGTAGAGCAGTTC 3' * | SEQ ID NO.10 |
| 5'CATTGGTGAAGGGCTCCATTTCCTTACAGCCGG 3' * | SEQ ID NO.11 |
| 5'CTTCCCTCAAACATGCTGTTTTCAATCATATTCCTGG 3'* | SEQ ID NO.12 |
| 5'TGGAGGAGGATGAAGAGGATTATG 3' * | SEQ ID NO.13 |
| 5'CTACGCTATGAACTCAGCCTACACCC 3' * | SEQ ID NO.14 |
| 5'GCCTACACCCGAACCTAACCTCGATGA 3' * | SEQ ID NO.15 |
| 5'CACAGGTCATAATATTTCAAGAGT 3' * | SEQ ID NO.16 |
| 5'AAGCCAAGAAAAACAAGCTGAGATCC 3' * | SEQ ID NO.17 |
| 5'ATAAGGGATGAGCAATAC 3' * | SEQ ID NO.18 |
| 5'CACCGCTTTATCAAATATGCCTAAG 3' * | SEQ ID NO.19 |
| 5'ATATGCCTAAGAAGTCATCAGTCGT 3' * | SEQ ID NO.20 |
| 5'CTACTTTGAAATTCAGTACCCACTT 3' * | SEQ ID NO.21 |
| 5'TTCAGTACCCACTTAAGAAATTGGAT 3' * | SEQ ID NO.22 |
| 5'TCACCTTCCGAGAGGAGACACTTCT 3' * | SEQ ID NO.23 |

### EXAMPLE 9 DEVELOPMENT OF An ASSAY FOR MONITORING THE ACTIVITY OF AT₄ RECEPTOR/IRAP

An assay may be used for the discovery of compounds which modulate the AT₄ receptor/IRAP activity. This assay may also be used for the discovery of compounds for the clinical applications listed herein. The assay uses an aminopeptidase substrate, a source of AT₄ receptor/IRAP - containing material, and a candidate modulator.

### 1. Providing AT₄ receptor/IRAP or AT₄ receptor/IRAP containing material

Potential sources of AT₄ receptor/IRAP-containing material include cell lines or biological tissues expressing appreciable amounts of AT₄ receptor/IRAP. Prokaryotic or eukaryotic cells expressing a transfected gene encoding AT₄ receptor/IRAP represent a recombinant source. In one embodiment of the invention, it is preferable to utilize material containing AT₄ receptor/IRAP which is identical to the form of AT₄ receptor/IRAP expressed in the brain. Our results indicate that the brain AT₄ receptor/IRAP is approximately 10kDa smaller than the one expressed in the periphery. The difference in size is thought to be due to differential glycosylation. Using the brain-derived AT₄ receptor/IRAP as a source for screening may enable the design of modulators that are specific to the brain form of AT₄ receptor/IRAP and therefore do not interact with the peripheral form. For example, this would be important for the design of drugs for the enhancement of memory and learning and for the treatment of dementia.

### 2. Monitoring the enzymatic activity of IRAP in the presence of a test substance

AT₄ receptor/IRAP-containing material is incubated with a synthetic aminopeptidase substrate, such as L-leucyl β-naphthylamide, and a test substance. Inhibition or enhancement of the rate of degradation of the synthetic substrate is a measure of the modulatory effect of the test substance.

For example, Hek293 cells stably transfected with a eukaryotic expression vector expressing the AT₄ receptor gene may be utilized. An example of this is the vector pcDNA-3 containing the cDNA encoding the full length AT₄ receptor. Two assay systems are used, one for each spliced variant of the human AT₄ receptor gene. The assay system involves the measurement of the rate of hydrolysis of L-leucyl β-naphthylamide by the AT₄ receptor. The rate of hydrolysis by the transfected cells is followed by recording the increase in fluorescence (excitation at 340nm, emission at 410nm) using a purpose-designed fluorimeter. Test compounds are added to the assay system, and their effects on the hydrolysis of L-leucyl β-naphthylamide are a direct indicator of their modulation of AT₄ receptor activity.

### EXAMPLE 10 PEPTIDE INHIBITORS OF IRAP/AT₄ RECEPTOR ACTIVITY

We believe that small peptides which bind specifically to the AT₄ receptor to mediate effects on learning and memory act by inhibiting the enzymatic activity of IRAP.

Crude membranes were prepared from HEK 293 cells stably transfected with pcDNA-3 containing the cDNA encoding the full length AT₄ receptor/IRAP, using the same method as that described for bovine adrenal membranes. The membranes were solubilised in 50 mM Tris-HCl containing 1% Triton-X100 at 4 °C for 16h. The IRAP enzymatic activity was determined by the hydrolysis of a synthetic substrate, L-leucine-β-naphthylamide, and monitored by the release of a fluorogenic product on a spectrophotometer set at excitation 340 nm and emission 410 nm. One hundred µM of the substrate was added to 10 µg of solubilised membranes, and the increase in fluorescence monitored for 10 min. The peptides Ang IV or LVVYPWTQRF (SEQ ID NO:2) (10 µm) were added, and the extent of inhibition of enzymatic activity determined.

In this assay, 10 mM of Ang IV or LVVYPWTQRF (SEQ ID NO:2) totally abolished the hydrolysis of L-leucine-β-naphthylamide by membranes containing high concentrations of IRAP, as shown in Figure 12a. This indicates that in addition to binding to IRAP, these peptides inhibit the catalytic activity of the enzyme.

All the AT₄ ligands tested inhibited the cleavage of the synthetic substrate, L-leucine-β-naphthylamide, by IRAP, with IC₅₀ values between 200 nM and 4 µM, as summarised in Table 1. The rank order of potency was Ang IV > Nle¹-Ang IV > LVV-hemorphin-7 > divalinal-Ang IV. In contrast, AVP, which was readily cleaved by IRAP, displayed an IC₅₀ > 40 µM. IRAP activity could also be inhibited by the aminopeptidase inhibitors, amastatin and bestatin, but only at concentrations much higher than observed for other aminopeptidases (25% inhibition of IRAP at 100 µM amastatin, 60% at 10 uM bestatin).

**Table 1 - Comparison of the apparent affinities of AT₄ ligands, as assessed by radioligand displacement and fluorescent substrate cleavage inhibition assays.**

| | IC₅₀ binding | IC₅₀ inhibition |
|---|---|---|
| | ¹²⁵I-Nle-Ang IV | leucine β-naphthylamide |
| Nle¹-Ang IV | 3.32 nM | 600 nM |
| Ang IV | 32.1 nM | 200 nM |
| LVV-H7 | 140 nM | 1. 5 µM |
| Divalinal -Ang IV | 845 nM | 4 µM |

Kinetic analysis was performed over a range of substrate concentrations (15.6 µM - 1 mM), in the absence and presence of Ang IV (0.25, 0.5, or 1 µM) or divalinal-Ang IV (2.5, 5, or 10 µM). Samples were assayed 2-3 times in triplicate, except controls, which were performed 5 times in triplicate. At the highest concentration of each inhibitor, cleavage of 15.6 µM L-leucine-β-naphthylamide was essentially undetectable; therefore these samples were omitted from analysis. Linear regression analysis of kinetic data expressed in double-reciprocal (Lineweaver-Burk) form resulted in R values greater than 0.99 for each curve. Inhibitor constants (K_{I}) for competitive inhibitors were calculated from the relationship IC₅₀ = Kᵢ(1 + [S]/Kₘ), where Kₘ for L-leucine-β-naphthylamide was determined from the kinetic experiments to be 32.3 µM.

The results of kinetic analysis of the inhibition of IRAP by Ang IV and divalinal-Ang IV are shown in Figure 12b. Both these peptides displayed competitive inhibition of IRAP, in that the apparent Kₘ for L-leucine-β-naphthylamide increased up to 14-fold with increasing inhibitor concentrations, with only minor changes in Vₘₐₓ (< 30% decrease from control value of 450 pmol/µg protein/hr). Kᵣ values for Ang IV and divalinal Ang IV were calculated to be 113 nM and 2.25 µM, respectively. Nle¹-Ang IV and LVV-H7 were also competitive inhibitors; their respective K_{I} values were 340 nM and 845 nM.

### EXAMPLE 11 DEVELOPMENT OF PEPTIDOMIMETIC AGENTS TO MODULATE THE ACTIVITY OF AT₄ RECEPTOR/IRAP

Therapeutic agents which modulate the enzyme are also developed on the basis of structure-function studies on peptides known to modulate AT₄ receptor/IRAP. These peptides include Ang IV, Nle¹-Ang IV, LVVYPWTQRF (SEQ ID NO:2) and the complexin fragment (KYLPGPLQ (SEQ ID NO:3)), known ligands of AT₄ receptor/IRAP such as oxytocin and vasopressin, and peptide fragments thereof, and known inhibitors of aminopeptidases, enabling the development of peptidomimetic and other compounds that modulate AT₄ receptor/IRAP activity. Structure-activity studies carried out on amino- and carboxy-terminal extensions and deletions of the peptides Ang IV and LVVYPWTQRF (SEQ ID NO:2) using the AT₄ radioreceptor assay and 125I-Ang IV revealed that the VYXX motif is important for binding to AT₄ receptor/IRAP, as shown in Figure 13. Lead peptidomimetic compounds based on this structural backbone are being developed.

### EXAMPLE 12 DEVELOPMENT OF A DIAGNOSTIC MARKER

During pregnancy AT₄ receptor/IRAP is highly expressed in the placenta. The protein is cleaved from the cell surface and circulates in the maternal serum. The serum levels of the AT₄ receptor/IRAP can be determined using a radioimmunoassay. Serum and/or CSF levels of AT₄/IRAP have the potential to be a diagnostic or prognostic marker for disorders related to the actions of AT₄ receptor/IRAP. Examples of these include central nervous system disorders, including cognitive, motor or sensory disorders such as Alzheimer's disease; motor neuron disease; cardiovascular disorders, including hypertension, congestive heart failure, cardiac hypertrophy, vasospastic disorders, and atherosclerosis; and premature or delayed labour.

### EXAMPLE 13 IDENTIFICATION OF THE ENDOGENOUS SUBSTRATE/S OF AT₄ RECEPTOR/IRAP

This example relates to a method of identifying and characterising peptides that are altered by the AT₄ receptor/IRAP system including the endogenous substrate of the enzyme, particularly in the brain.

We propose that one mode of action of modulators of AT₄ receptor/IRAP is via extending the half-life of peptide substrates, by inhibiting their degradation by AT₄ receptor/IRAP. The endogenous substrates for AT₄ receptor/IRAP are identified, and their relationship to the clinical disorders and biological effects of modulators of the AT₄ receptor/IRAP system is determined. Therefore another approach for the treatment of these disorders is to identify the targets of the AT₄ receptor/IRAP system, and to test these compounds directly, using bioassays such as memory, learning, wound healing, neuronal regeneration etc.

Based on the structure of known peptide modulators for AT₄ receptor/IRAP a panel of peptides is designed, synthesise and tested as potential peptide substrates for AT₄ receptor/IRAP. Based on the ability of the peptides to be altered by the AT₄ receptor/IRAP system, selective intervention such as specific blockade at the receptor level, is tested in biological systems where Ang IV and other modulators of AT₄ receptor/IRAP activity have a known effect. Therefore we can identify the peptide substrates which are directly involved in the biological systems affected by the AT₄ receptor/IRAP system.

For example, a peptide is incubated with AT₄ receptor/IRAP containing material and its rate of degradation measured by HPLC. If the peptide proved to be a substrate in the AT₄ receptor/IRAP system then potentially effects of modulators of AT₄ receptor/IRAP observed in vivo and in vitro may be mediated by the thus-identified substrate peptide. To confirm this hypothesis, the substrate peptide is tested in biological systems where effects of Ang IV and its analogues have been observed. Alternatively, if the peptide is known to be part of an already characterised system, antagonists to the corresponding receptor are utilized to define the observed effects with Ang IV and other modulators of AT₄ receptor/IRAP activity.

Two candidate substrates for AT₄ receptor/IRAP in the brain are arginine-vasopressin (Herbst et al, 1997) and oxytocin (Rogi et al, 1996), two structurally related peptides which occur in relatively high abundance in the brain and have been shown to be cleaved by IRAP *in vitro.* These peptides have been shown to have effects on memory in certain behavioural paradigms, and thus present two potential substrates for IRAP in neurones. Other candidate substrates of IRAP include somatostatin, angiotensin III, Lys-bradykinin, neurotensin, metenkephalin, dynorphin A, neurokinin A, neuromedin B, and cholecystokinin 8 (Matsumoto et al, 2001), all of which are neuropeptides which play important roles in the central nervous system.

Each potential peptide substrate (30 µg) was incubated at 37°C in TBS with 10 µg solubilized membrane protein from hIRAP transfected-HEK 293T cells. At each time point (0, 0.5, 1, 2, 4 & 6 hr), an aliquot containing 5 µg peptide was removed, and the reaction stopped by addition of 4 volumes methanol/1% trifluoroacetic acid (TFA). Samples were dried on a centrifugal vacuum evaporator prior to HPLC analysis with on-line mass spectrometric detector. Samples were loaded on to a Zorbax Eclipse C18 column (maintained at 50°C) in 1.8% acetonitrile/0.1% TFA/0.02% acetic acid at 0.15 ml/min, and eluted with a 30 minute linear gradient to 60% acetonitrile/0.1% TFA. Fragments were identified following mass spectral analysis, using Agilent ChemStation deconvolution software. The results are summarised in Table 2.

Among the range of potential substrates tested, arginine-vasopressin (AVP), oxytocin (OXY) and met-enkephalin were the most rapidly cleaved by IRAP. Both Cyt⁶-AVP(2-9) and Cyt⁶-AVP(3-9) were generated by IRAP, but only the first peptide bond of OXY was cleaved. Similarly, although the first residue (Tyr¹) of met-enkephalin was quickly removed, only a small amount of desTyr¹Gly²-met-enkephalin was detected after 6 hours. This contrasts with the longer opioid peptide α-neo-endorphin (10 residues), in which at least three N-terminal residues were cleaved, although less rapidly than met-enkephalin. Furthermore, α-endorphin, which is 16 residues long, with the first five residues identical to met-enkephalin, was not cleaved at all, suggesting that peptide chain length is an important determinant of substrate specificity and efficiency. The only other peptide to be cleaved by IRAP was neurotensin, despite the presence of a pyroglutamyl residue at the N-terminus in this peptide.

Among the angiotensin peptides, no cleavage was observed for Ang I, Ang II, or divalinal-Ang IV. No cleavage of the other peptides tested (GnRH, acetylated AVP, and [pGlu⁴Cyt⁶]AVP(4-9)) was observed.

**Table 2 - Degradation of Ang IV-related and other peptides by IRAP expressed in HEK 293T cells.**

| Peptide | Products Identified | Substrate disappearance over 4 hr |
|---|---|---|
| AVP | YFQNCytPRG-NH₂ FQNCytPRG-NH₂ | 58% |
| OXY | YIQNCytPLG-NH₂ | 37% |
| Met-enkephalin | GGFM | 65% |
| Neurotensin | LYENKPRRPYIL | 22% |
| Ang IV | YIHPF | 15% (= UT) |
| Nle¹-Ang IV | YIHPF | 16% (= UT) |
| LVVYPWTQRF | VVYPWTQRF | 14% (= UT) |
| Ang III | RVYIHP | 7% (= UT) |
| α-neo-Endorphin | GGFLRKYPK, GFLRKYPK, FLRKYPK | 9% |
| Divalinal Ang IV | None | None |
| Ang I | None | None |
| Ang II | None | None |
| Sar¹Ile⁸-Ang II | None | None |
| GnRH | None | None |
| AcAVP | None | None |
| pGlu⁴Cyt⁶AVP | None | None |
| α-Endorphin | None | None |

| | | |
|---|---|---|
| UT : untransfected cells used as the control | | |

In adipocytes and skeletal muscles, IRAP is found in the same vesicles as GLUT4, the insulin-dependent glucose transporter. As in the case of GLUT4, IRAP is redistributed to the cell surface in response to insulin or insulin-like growth factors. We postulate that IRAP may serve as a negative feedback control for GLUT4 translocation to the cell surface and hence glucose transport into the cell by regulating the levels the insulin and insulin-like growth factors on the cell surface and also in the GLUT4 vesicles. We are investigating the hydrolysis of insulin IGF-I and IGF-II by IRAP using HPLC with C18 reverse phase columns.

### EXAMPLE 15 EFFECTS OF INHIBITORS/SUBSTRATES OF AT₄ RECEPTOR/IRAP ON ANIMAL MODELS OF MEMORY AND LEARNING

Previous studies have shown that binding of peptide agonists to the AT₄ receptor in normal animals led to improved performance of memory tasks in both passive avoidance and spatial learning paradigms, and reverses memory deficits induced chemically by muscarinic antagonist or mechanically by knife-cut lesions. The initial effects were observed in the passive avoidance paradigm in rats where an intracerebroventricular dose (1 nmol) of Ang IV increased the latency in re-entering the dark chamber after an aversive stimulus (Braszko et al, 1988, Wright et al, 1993, Wright et al, 1996). More recent studies revealed that a more stable analogue of angiotensin IV, Nle-angiotensin IV, given acutely into the lateral ventricles, reversed the memory deficits induced by the muscarinic receptor antagonist, scopolamine in a spatial learning paradigm (Pederson et al, 1998). Chronic infusion (6 days) of this analogue into the lateral ventricle of rats at a dose of between 0.1 and 0.5 nmol/h enhanced performance in the swim maze task. This effect was blocked by divalinal angiotensin IV, suggesting that this was an angiotensin AT₄ receptor mediated effect (Wright et al, 1999). In this swim maze paradigm, memory deficits induced by bilateral perforant pathway lesion can be reversed by an acute dose (1 nmol) of another AT₄ receptor agonist, Norleucinal angiotensin IV (Wright et al, 1999).

In the context of this invention, modulation of the activity of the aminopeptidase AT₄ receptor/IRAP by a number of peptides known to be AT₄ receptor agonists results in enhancement of learning and memory in normal and amnesic animals.

### EXAMPLE 16 EFFECTS OF MODULATION OF AT₄ RECEPTOR/IRAP ON SPATIAL AND LEARNING MEMORY IN NORMAL RATS

Normal male Sprague-Dawley rats were implanted with cannulas in the lateral ventricles and allowed to recover for at least three days. The animals were subjected to a modification of the Barnes Circular Maze test (Barnes, 1979). The maze comprised a raised rotatable white circular platform of diameter 1.2m, with 18 evenly spaced holes in the periphery (diameter 0.09m). An escape tunnel comprising a black box of internal diameter of 0.16m width, 0.29m length and 0.09m depth was positioned immediately beneath a peripheral hole. The maze was positioned in a large room, 6 x 4.5m, both naturally and fluorescently lit with visual cues (e.g. A4 sized posters of abstract patterns) placed on stands at various positions around the maze at a distance of 0.5m from the platform.

For each trial, the animal was placed on the maze platform under the starting chamber, which is a cylindrical chamber located in the centre of the platform and can lowered or raised, and left for 20s. While the animal was under the chamber, the floodlights were switched on. Following the twenty seconds disorientation period the chamber was raised, and the trial was begun. The animals were then allowed 240s in which to find and utilize the escape tunnel. Following 20s of light alone, the auditory buzzer was switched on, and these aversive stimuli were continued throughout the remaining time period (220s) or until the escape tunnel was found and utilized. Each rat received three consecutive trials per day over ten days. On the first day of the testing period, each rat was individually taken into the testing room in its cage, and placed directly into the escape tunnel for a 2min familiarization period. The rat was then replaced into its cage for approximately one minute, after which the animal was injected with peptide or artificial cerebrospinal fluid and then replaced into its cage for a further 5min. Three consecutive trials were then carried out, with a recovery period of 2min between each trial in its home cage.

The escape tunnel and platform were cleaned to eliminate the possible use of olfactory cues in the next trial. The platform was then rotated so that a different peripheral hole was placed above the escape tunnel although the spatial location of the escape tunnel is kept constant for each animal. On subsequent days (days 2 to 10), the familiarization period prior to the three trials was eliminated from the protocol.

In the Barnes maze, treatment with 100pM or 1nM of Nle¹-Ang IV or LVVYPWTQRF (SEQ ID NO:2), both competitive inhibitors of IRAP, decreased the time taken to achieve learner criteria in this paradigm. The results are illustrated in Figures 14a and 14b. The learner criterion is dependent on the number of errors made per trial and the time taken to reach the escape tunnel for each trial. Control animals treated with artificial cerebrospinal fluid took 7 days to achieve learner criteria, whereas animals treated with Nle¹-Ang IV or LVVYPWTQRF (SEQ ID NO:2) at a concentration of either 100pM or 1nM LVVYPWTQRF (SEQ ID NO:2)managed this in 3-4 days (Figure 14a). Moreover, during each trial, even from day 1 of testing, the peptide-treated animals consistently made fewer errors per trial, and travelled shorter distances to find the escape tunnel than the artificial cerebrospinal fluid control. This observation strongly indicates that the two peptides tested not only improved memory, but also enhanced the ability of the animals to learn.

### EXAMPLE 17 EFFECT OF MODULATION OF AT₄ RECEPTOR/IRAP ACTIVITY ON SPATIAL MEMORY IN NORMAL MICE

The effect of modulation of AT₄ receptor/IRAP activity on spatial memory in normal mice was then tested using the Y-maze test. This test is another behavioural paradigm which measures spatial memory performance, and exploits the natural instinct of rodents to explore novel environments. This test is less stressful than other behavioural paradigms, including Morris water maze and passive avoidance tasks, which can modify the motivational and emotional status of the animals.

The Y maze consists of three identical alleys (30cm in length, 10cm in width and 17cm in height) with the three arms separated by 120° angles. To minimize stress, the maze is located in a sound-attenuated room under dim illumination, with the floor of the maze covered with sawdust. After each trial, the sawdust is mixed to eliminate olfactory cues. For spatial orientation, visual cues are placed on the walls of the testing room.

The test consisted of two trials, separated by a time interval known as the intertrial interval. During the acquisition trial, the animals were placed at the distal end of one arm, their heads pointing away from the centre of the maze. The mice were allowed to visit only two accessible arms of the maze for 3 minutes. At the end of the acquisition trial, the mice were replaced in their cages for the intertrial interval. During the retention trial, the animals had access to all three arms for 5 minutes. The number and duration of time spent in the novel arm were documented.

A shorter intertrial interval (2 hours) separating the acquisition phase from the retention phase enables amnesiant effects to be detected. Control mice still remember the location of the novel arm and will preferentially spend more time (45-50% of time) in that arm. A longer intertrial interval (6 hours) results in the animals not remembering the location of the novel arm and hence spending equal amount of time in all three arms.

Mice were surgically implanted with an infusion cannula in the dorsal third ventricle. The cannula was connected to Alzet osmotic minipumps by tygon tubing (5-6cm in length and 0.3mm in diameter). The minipumps were placed in the dorsal subcutaneous space in the neck. The minipumps were filled with artificial cerebrospinal fluid for 1 week, and delivered the fluid at a constant flow rate of 0.5µl/h. At the end of the first week, the minipumps were replaced under light anaesthesia with another minipump containing either artificial cerebrospinal fluid or LVVYPWTQRF (SEQ ID NO:2). The peptide was delivered at a rate of 0.3pmol/h.

Each mouse was tested three times, and each test was separated by at least three days. The longer intertrial interval regime was adopted to test for memory-enhancement potential of the peptide. The first test was performed prior to implantation of the minipumps with treatment, and hence constituted measurement of the basal memory potential of the animals. The second and third tests were carried out on days 3 and 6 of treatment delivery. The results are summarised in Figure 15.

After three days of treatment, mice which received LVVYPWTQRF (SEQ ID NO:2) spent more time in the novel arm (between 50-60% of time) compared to control mice (approximately 33% of time), indicating that during that time period, LVVYPWTQRF (SEQ ID NO:2) enhanced the spatial memory in this paradigm. However, after 6 days mice from both treatment groups were indistinguishable from each other. These results indicate that prolonged treatment with LVVYPWTQRF (SEQ ID NO:2) is detrimental to spatial memory, and may be the result of excessive accumulation of the peptide in the hippocampus.

### EXAMPLE 18 EFFECT OF MODULATION OF AT₄ RECEPTOR/IRAP ACTIVITY ON SPATIAL LEARNING AND MEMORY IN AMNESIC ANIMALS

The effect of modulation of AT₄ receptor/IRAP activity on spatial learning and memory in amnesic animals was also assessed using the Morris water maze test. The Morris test has been used extensively to test for spatial memory. The hippocampus, which contains high levels of AT₄ receptors/IR.AP, is thought to play an important role in processing this form of memory.

Scopolamine, a muscarinic receptor antagonist, has been used to induce amnesia in a number of behavioural paradigms of memory and learning. This compound has been shown to block the trial-to-trial decrease in latency of this task, and this effect appeared to be due to impairment of short-term memory. The effect of AT₄ receptor/IRAP ligands on scopolamine-induced amnesia in a spatial memory task was tested in the Morris water maze.

The maze consisted of a circular tank, 1.6m in diameter by 0.6m in height, containing water to a depth of 30cm. The room contained visual cues placed 40 cm from the edge of the tank. The rats were subjected to two days of familiarisation to the task, during which the escape pedestal is visible to the animals. During the acquisition phase, for the following 5 days, the water in the tank was rendered opaque, and the escape platform was submerged under the surface of the water. The animals had to rely on the visual cues around the room to find the submerged escape pedestal. The latency of the animal in finding the pedestal during the acquisition phase was taken as a measure of the spatial memory performance of the animal.

Rats were surgically implanted with cannulae in their lateral ventricles and allowed two to three days of recovery. During the familiarisation trials on days 1 and 2, the animals were placed in the water facing the wall of the tank from different starting positions equidistant from the escape pedestal. When the animals located and mounted the pedestal, they were allowed 30 seconds on the pedestal before the commencement of the next trial. Four consecutive trials were carried out on each animal on each day, and the mean latency period before the animal reached the pedestal was plotted.

For the acquisition trials, from days 3-7, the animals were randomly divided into three groups:
a) saline control,
b) scopolamine followed by saline treatment, and
c) scopolamine followed by LVVYPWTQRF (SEQ ID NO:2) treatment.

The scopolamine-treated animals were given 70nmole of the compound into the lateral ventricles 30min before the commencement of the trials for each day. The peptide (1nmole) or saline was given 5min before the trial began. The pedestal, which remained in a constant position for each animal, was submerged for this phase, and the mean latency for each animal in locating the pedestal was documented for each day. The drug treatments were withdrawn on the eighth day, the pedestal once again made visible, and latency documented. The results are shown in Figure 16.

Intracerebroventricular infusion of 70nmole of scopolamine significantly increased the latency of the animals in locating the submerged pedestal, indicating a deficit in short-term memory. Rats treated with LVVYPWTQRF (SEQ ID NO:2) reversed the scopolamine-induced latency, and this group of rats was indistinguishable from saline-treated controls. Withdrawal of treatment on day 8 brought the latency of the scopolamine-treated group back to control levels, indicating that the effect of scopolamine was reversible, and that the latency was primarily due to the muscarinic receptor antagonist.

### EXAMPLE 19 EFFECT OF MODULATION OF AT₄ RECEPTOR/IRAP ACTIVITY ON AVERSIVE CONDITIONING BEHAVIOUR IN AMNESIC ANIMALS

Passive avoidance trials were then used to test the effect of modulation of AT₄ receptor/IRAP activity on aversive conditioning behaviour in amnesic animals. The passive avoidance task involves aversive conditioning behaviour to measure facilitation of memory retention and retrieval. The testing was carried out in an apparatus that consisted of a light and a dark compartment separated by a guillotine door. The floor of the dark compartment contained an electrified grid.

Rats were surgically implanted with chronic cannulae in the lateral ventricle. On conditioning day, the animals were habituated in the dark compartment for 5min with the guillotine door closed. The animals were then placed in their home cages for 5min before being placed in the light compartment with the guillotine door opened. The latency to enter the dark compartment with all four feet was documented. When the rats entered the dark compartment within 20s of being placed in the light compartment, they were randomly divided into four treatment groups. The treatment groups were:
(1) saline followed by saline;
(2) saline followed by 1.0nmole LVVYPWTQRF (SEQ ID NO:2);
(3) 70nmole scopolamine followed by saline; and
(4) 70nmole scopolamine followed by 1.0nmole LVVYPWTQRF (SEQ ID NO:2).

All of these were administered into the cerebral ventricles 30min and 5min before the acquisition trial. During this trial, the guillotine door was closed once the animal entered the dark compartment, and the rat then received a low-level electric shock (0.2mA) via the grid floor. The animal was then returned to its home cage for 24h before being tested once daily for the next four days and the latency periods to re-enter the dark compartment were taken as a measure of the ability of the animals to remember the aversive stimuli. The results are shown in Figure 17.

Control rats displayed high latency periods in entering the dark compartment, due to the successful conditioning, whereas rats treated with scopolamine displayed memory deficits, as indicated by the much lower latency periods. The performance of rats which received LVVYPWTQRF (SEQ ID NO:2) after scopolamine was not significantly different from controls indicating that the decapeptide completely reversed the amnesia induced by scopolamine. Rats which received LVVYPWTQRF (SEQ ID NO:2) alone displayed latency periods that were comparable to the scopolamine-treated group. This suggests that at high doses, LVVYPWTQRF (SEQ ID NO:2) is detrimental to learning, as was observed in the Y maze paradigm.

### EXAMPLE 20 MECHANISM OF MEMORY/LEARNING ENHANCEMENT OF THE IRAP/AT₄ RECEPTOR SYSTEM

Acetylcholine is a major neurotransmitter involved in the processing of cognitive function, since anti-cholinergic drugs induce memory deficit and confusion. In Alzheimer's disease, neuronal loss has been reported in cholinergic-rich areas, particularly in the septohippocampal pathway. Angiotensin AT₄ receptors have been found in high abundance in the basal nucleus of Meynert, in the CA2 and dentate gyrus of the hippocampus, and in somatic and autonomic preganglionic motoneurones of the monkey brain. This pattern of receptor distribution closely resembles that of cholinergic neurones and their projections, and suggests that the AT₄ receptors may be associated with cholinergic pathways centrally. Moreover, as shown in Example 19, LVV-haemorphin-7 can reverse the learning deficit induced by scopolamine (a muscarinic receptor antagonist). We therefore postulated that LVVYPWTQRF (SEQ ID NO:2) can modulate acetylcholine release from the septohippocampal neurones via the AT₄ receptors. In the light of:
(a) our discovery that IRAP is the AT₄ receptor;
(b) the possibility that IGF or insulin is a substrate for IRAP; and
(c) the fact that glucose has been shown to facilitate acetylcholine synthesis and release we believe that the AT₄ receptor/IRAP may be regulating glucose uptake into the hippocampus resulting in an increase in acetylcholine release.

To determine the effect of IRAP inhibitors an acetylcholine release for rat hippocampal slices *in vitro,* rats were anaesthetized with sodium pentobarbitone, decapitated and the brain rapidly extracted and put on ice. The two sides of the hippocampus were then dissected, and stored in Krebs buffer on ice. The tissues were sectioned into 400µm slices using a McIlwain slicer, and incubated with 10µM of ³H-choline in Krebs buffer for 20min at 37°C. Following the incubation, the sections were transferred to Brandel superfusion chambers filled with carbogenated Krebs buffer, pH7.0 containing 50mM hemicholinium HC-3 to prevent choline reuptake. The slices were washed for 60min at 37°C with Krebs buffer at a flow rate of 500µl/min. Superfusate samples were collected at 5min intervals for the duration of the experiment. After a stable baseline was established, the hippocampal slices were depolarised by the addition of 30mM KCl to the Krebs buffer and infused for 4min (stimulation period S1). The tissue was then allowed to recover for 25min before the next stimulation period (S2). To test the effect of LVVYPWTQRF (SEQ ID NO:2) or Ang IV on the evoked release of ³H-choline, the peptides were added to the hippocampal slices 15min prior to and 5min during the second period of stimulation. At the end of the experiment, tissues were collected and solubilized with 2ml of soluene to determine the counts remaining in the hippocampal slices. Fractional ³H-choline release, expressed as a ratio of S2/S1, was then calculated for each of the different peptides at the different doses.

In rat hippocampal slices, LVVYPWTQRF (SEQ ID NO: 2) significantly and dose-dependently increased potassium-evoked ³H-choline release, with a maximum effect of 180% of control observed at 10⁻⁷M, as shown in Figure 18. Similarly, Ang IV, also significantly and dose-dependently increased potassium-evoked ³H-choline release with a smaller maximum effect of 150% of control observed at 10⁻⁷M, as shown in Figure 18.

### EXAMPLE 21 EFFECT OF AT₄ RECEPTOR/IRAP LIGANDS ON AND MAP KINASE, CREB AND ELK-1 PHOSPHORYLATION

Long-term neuronal plasticity and memory consolidation require de novo gene expression which is thought to be mediated by the activation of certain transcription factors, such as the cAMP response element binding protein (CREB) and Elk-1(a transcriptional regulator of serum response element-driven genes), via the ERK/mitogen activated protein kinase (MAPK) signalling cascade. The ERK/MAPK cascade involves three tiers of kinases which are sequentially activated by phosphorylation. The MAP kinase kinase kinases, Raf-1 and B-Raf, activate the MAP kinase kinase, MEK, by serine/threonine phosphorylation; MEK then activates p44 MAPK and p42 MAPK, by threonine and tyrosine phosphorylation. Phosphorylation of these MAPKs is thought to bring about their translocation to the nucleus, where they alter gene expression via activation of CREB and Elk-1. MAPK is also thought to regulate the voltage-dependent activation of the potassium channel, Kv4.2, which plays a key role in regulating calcium influx. The MAPK cascade is activated by multiple signals, including activation of receptor tyrosine kinases, synaptic activity and increases in intracellular Ca2+ and cAMP, and is thus capable of integrating several different signals into a graded response.

The mechanism(s) by which IRAP/AT₄ substrates enhance memory may involve modulation of the MAPK cascade. For instance, inhibition of the aminopeptidase activity of IRAP/AT₄ by ligand binding might bring about the accumulation of a neuropeptide that activates the MAPK cascade via an interaction with a cell surface receptor.

The potential of IRAP/AT₄ ligands to activate MAPK or CREB was initially investigated on rat hippocampal slices. Rats were killed by decapitation and the hippocampi removed, four hundred µm thick slices cut and incubated in aCSF. After four hours of stabilisation, the slices were treated with 10⁻⁸ M Nle¹Ang IV for 30 min, or left untreated. Slices were then lysed in 200 µl of ice-cold lysis buffer (10 mM Tris-HCl pH 7.5, 50 mM NaCl, 1% triton X-100, 30 mM sodium pyrophosphate, 50 mM NaF, 5 µM ZnCl₂, 0.1 mM sodium orthovanadate, 1mM DTT, 5 nM okadaic acid, 1 x Boehringer Protease inhibitor cocktail) and centrifuged at full speed. The lysates were then heated at 95°C for 10 min in 6 x SDS loading buffer and the proteins (30 µg per lane) resolved on 7.5% SDS-poylacrylamide gels. After transfer of proteins to PVDF membranes, blots were probed overnight at 4°C with anti phospho-p44/42 MAP kinase (Thr202/Tyr204) antibody and detected by chemiluminescence using ECL blotting detection reagents and a Fuji LAS-1000.

A single dose of 10⁻⁸M Nle¹-Ang IV induced phosphorylation of ERK1/2 in rat hippocampal slices after 30 min, as shown in Figure 19a. This effect was observed throughout the length of the hippocampus.

Enhanced activation of the MAPK cascade by IRAP/AT₄ ligands may only occur during learning. Therefore, animals treated IRAP/AT₄ ligands or vehicle control are subjected to varying numbers of learning trials (as described in the previous example), before analysis of the levels of phospho-MAPK phospho-CREB and phospho-Elk-1 in their hippocampi as described above.

Alternatively, binding of ligands to IRAP might generate an intracellular signal which activates the MAPK cascade. The ability of AT₄ ligands to stimulate MAPK phosphorylation directly was investigated in HEK293 cells transfected with human IRAP. HEK293 cells were transfected with pCI-IRAP or with empty vector, plated on to 12-well plates at a density of 60,000 cells/well and grown in complete medium containing 0.2% fetal calf serum. The cells were then treated with 10⁻⁶, 10⁻⁹ or 10⁻¹¹ M Nle¹-Ang IV for 5,30 or 120 min. After peptide treatment, the medium was removed, the cells washed in phosphate-buffered saline and solubilised in 100µl of 1xSDS loading buffer. The solubilised samples were then heated at 100°C for 5 min, centrifuged and 50µl of the supernatant run on a 12% SDS-PAGE. The proteins were then transferred on to nitrocellulose membranes and incubated with phospho-MAPK (p44/42) antibodies and visualised with HRP-conjugated secondary antibody, using the ECL-plus western blot detection system.

Nle¹-Ang IV at concentrations of 0.001, 1 and 10 µM, induced the phosphorylation of ERK1/2 within 5 min of peptide treatment, and the phosphoERK persisted for 2 h, as shown in Figure 19b. The largest response was observed at 0.001 µM of Nle¹-Ang IV.

### EXAMPLE 22 IRAP COLOCALISES WITH GLUT4 IN NEURONES - COMPLEMENTARY ROLE IN THE REGULATION OF GLUCOSE UPTAKE IN NEURONAL CELLS

We have also investigated the role of AT₄ receptor/IRAP in the regulation of glucose uptake into neuronal cells. GLUT4 was initially found predominantly in adipocytes and skeletal muscles, where it is highly expressed in vesicles. Activation of the insulin receptor by insulin or insulin-like growth factors in these cells results in the translocation of GLUT4 vesicles to the plasma-membrane. This insulin-dependent glucose transporter was recently detected in the brain and in developing mouse embryos. In the brain, the distribution of GLUT4 protein and mRNA closely parallels the location of the AT₄ receptor, as demonstrated by ¹²⁵I-Ang IV or ¹²⁵I - Nle Ang IV binding. Since IRAP is the AT₄ receptor, we proposed that, as in adipocytes and skeletal muscles, GLUT4 is co-localised with IRAP in neurones, and subserves a similar role.

We investigated the cellular localisation of IRAP, using an antibody raised in rabbits against a GST fusion protein containing the cytoplasmic tail of murine IRAP. We also investigated the cellular localisation of GLUT4, using a monoclonal antibody against mouse GLUT4.

Mice (C57/Black females) were anaesthetised with an intraperitoneal injection of pentobarbitone, and perfused with phosphate-buffered saline (PBS) followed by 4% PFA, 2% glutaraldehyde in phosphate buffer (pH 7.4). The brains were taken and stored in the fixative overnight at 4°C, then snap-frozen in isopentane cooled with dry ice. Forty micron thick sections were cut on a cryostat and collected into PBS. The free-floating sections were then incubated for one hour at room temperature in PBS with normal horse serum (10%) and Triton X-100 (0.3%). The sections were transferred to a blocking solution (NEN TSA kit) for 30min before incubation with the primary antibodies. Both antibodies (rabbit anti-IRAP and mouse anti-NeuN or rabbit anti-IRAP and mouse anti-GLUT4) were diluted 1:200 in PBS with 2% normal horse serum and 0.3% Triton X-100. The sections were incubated for 48h at 4°C.

Following incubation with the primary antibodies, the sections were washed three times in PBS and incubated for an hour at room temperature in anti-rabbit and anti-mouse IgG secondary antibodies conjugated to Texas Red (anti-rabbit) and FITC (anti-mouse). These antibodies were diluted in PBS with 2% normal horse serum. The sections were washed three times in PBS, mounted onto gelatine-coated slides and a coverslip was added with DAKO fluorescent mounting medium. The results are shown in Figure 20.

We found, using immunohistochemical staining of mouse hippocampus with an IRAP antibody, that IRAP was present in all pyramidal cells of the CA1-CA3 region and the Purkinje cells of the cerebellum. Moreover, all cells which were immunopositive for IRAP also contained GLUT4-positive immunoreactivity. However, the distribution of IRAP in the neurones appeared punctate, and occurred throughout the cell body and its processes, whereas the GLUT4 positive staining was perinuclear and therefore not in the same cellular compartment as IRAP (Figure 20). The intracellularly distributions of the two proteins did not reflect what is observed in adipocyte and skeletal muscles. We believe that in brain, IRAP may have wider roles in the regulation of local neuropeptide concentration, in contrast to IRAP in adipose tissue and skeletal muscle, where the enzyme coexists with GLUT4, and may be integrally involved in insulin-regulated glucose transport. However, it is still possible that in neurones, IRAP is involved in the regulation of the levels of insulin or insulin-like growth factors by enzymatic cleavage of these peptides, under certain physiological conditions such as during neural activation or pathophysiological states such as cerebral ischemia. Under these conditions, IRAP may play a role in the regulation of glucose uptake in neurones.

### EXAMPLE 23 EFFECT OF MODULATORS OF IRAP/AT₄ RECEPTOR ACTIVITY ON GLUCOSE UPTAKE INTO NEURONAL CELLS

Glucose has been found to improve memory in animals and humans, and several studies have shown that the memory-enhancing effects of glucose may not be due solely to its role in providing fuel for brain cells (Wickelgren, 1998). Glucose has been shown to facilitate acetylcholine synthesis (Kopf et al, 2001) and release (Kopf et al, 2001). Accordingly an investigation of AT₄ receptor/IRAP activity on glucose uptake in the hippocampus is undertaken.

Male Sprague-Dawley rats are surgically implanted with chronic cannulae in their lateral ventricles and allowed to recover for at least three days. To evaluate the effects of Ang IV or LVVYPWTQRF (SEQ ID NO:2) on hippocampal glucose uptake, the rats are infused with 1nmole of either peptide over a period of 10min. Immediately after the infusion of peptide, the rats are injected with 2-deoxy-D-[1-¹⁴C]glucose into the peritoneal cavity at a dose of 1µCi/g. The rats are killed 45min later, the brains extracted and snap-frozen and sectioned at 14µm thickness and exposed to Hyperfilm βmax.

Glucose utilisation is also measured on synaptosomes prepared from hippocampus. Synaptosomes are isolated from homogenised rat hippocampus using the Ficoll gradient centrifugation technique. The synaptosomes are washed in glucose-free HEPES buffer, then incubated at 37°C for 5min in the same solution containing 0.5mmole glucose. The synaptosomes are loaded with oxy-D-glucose,2-[1,2-³H(N)] at a concentration of 1µCi/ml. Uptake is assayed in the absence or presence of Ang IV or LVVYPWTQRF (SEQ ID NO:2) at concentrations of 1nM to 1µM. To terminate the experiments, the samples are centrifuged at 16000g for 30s, the supernatant discarded and the pellet rinsed twice with ice-cold HEPES buffer. The radioactivity retained is measured by scintillation counter, and expressed per mg protein. Hexokinase I activity, which is responsible for glucose-6 phosphorylation and essential for neuronal glucose utilisation, is also determined on the synaptosomal preparations, since a decrease in glucose utilisation is reflected by reduced hexokinase activity. Hexokinase activity is assayed on 0.2mg of freshly prepared synaptosomes suspended in MOPS-Tris buffer containing 0.1% Triton X-100, 8mM MgCl₂, 0.4M NADP⁺, 5mM D-glucose, 5 units of glucose-6-phosphate dehydrogenase and 1mM ATP in the absence or presence of Ang IV or LVVYPWTQRF (SEQ ID NO:2). The linear rate of NADP⁺ reduction is monitored on a spectrophotometer at 340nm absorbance and expressed as nM of product formed per mg protein.

We postulate that the modulation of AT₄ receptor/IRAP activity by Ang IV or LVVYPWTQRF (SEQ ID NO:2) results in an increase in glucose uptake in the hippocampus. This may be part of the mechanism via which the two peptides act to produce the effects on acetylcholine release and facilitation of memory.

The effect of IRAP inhibitors on the regulation of glucose uptake was determined in 3T3L1 cells. 3T3L1 fibroblasts were differentiated by the addition of DMEM containing 5% fetal calf serum, 4 µg/ml insulin, 0.25 mM dexamethasone and 0.5 mM 3-isobutyl-1-methyl-xanthine. After 7-14 days, more than 90% of the fibroblasts were differentiated into mature adipocytes. These cells were then placed in DMEM containing 25 mM glucose, 0.1% bovine serum albumin, washed in HEPES buffer and then treated with insulin (0.7 or 70 nM) or buffer with or without concurrent treatment with either 10⁻⁷ or 10⁻⁶ M Nle¹-Ang IV for 30 min. Glucose uptake was determined by incubation on ice for 5 min with 100 µM 2-deoxyglucose containing 1.0 µCi of 2- [³H] deoxyglucose. The reaction was stopped after 10 min by washing the cells in PBS. The cells were then solubilized in 0.5 N NaOH and counted.

Both concentrations of Nle¹-Ang IV increased basal glucose uptake two-fold : untreated (3.3 ± 0.67 pmol/min/well) compared to 10⁻⁷ M Nle¹-Ang IV (7.2 ± 1.79 pmol/min/well) and 10⁻⁶ M Nle¹-Ang IV (7.6 ± 1.21 pmol/min/well). Under insulin stimulation, glucose uptake increased to 13.5 ± 2.6 pmol/min/well for 0.7 nM insulin and 19.7 ± 1.18 pmol/min/well for 70 nM insulin. The effect of Nle¹-Ang IV on insulin-stimulated glucose uptake was not as marked. For both the concentrations of insulin, treatment with 10⁻⁷ M Nle¹-A IV did not significantly increase glucose uptake (12.2 + 2.29 pmol/min/well for 0.7 nM insulin and 22.99 + 2.8 pmol/min/well for 70 nM insulin) whereas treatment with 10⁻⁶ M Nle¹-Ang IV did (18.3 + 2.39 pmol/min/well for 0.7 nM insulin and 23.02 + 1.52 pmol/min/well for 70 nM insulin).

### EXAMPLE 24 EFFECT OF MODULATORS OF IRAP/AT₄ RECEPTOR ACTIVITY ON TROPHIC EFFECTS OF INSULIN OR INSULIN-LIKE GROWTH FACTOR

In addition to the role of insulin and insulin-like growth factor in chaperoning glucose into neurones via translocation of the GLUT4 vesicles, these two polypeptides could have other roles in the brain which may lead to facilitation of memory. Insulin and IGF-I have been shown to stimulate neuronal growth and promote arborisation of dendrites (Bondy, 1991, Wickelgren, 1998, O'Kusky et al, 2000). Some of the memory-enhancing effects of Ang IV and LVVYPWTQRF (SEQ ID NO:2), particularly with chronic infusions, may be the result of prolonging the half-life of insulin or IGF in the brain, resulting in an increase in neurogenesis or synaptogenesis.

This ability can be assessed by measuring neurogenesis in the hippocampus of rats subjected to the Barnes maze paradigm in the presence and absence of Nle-Ang IV or LVVYPWTQRF (SEQ ID NO:2), using a marker of dividing cells, 5-bromo-2'-deoxyuridine (BrdU). Male Sprague-Dawley rats are implanted with chronic intracerebroventricular cannulae and allowed two to three days recovery before the commencement of the experiment. The rats are divided into three groups:
1) Naive animals receiving no training on the Barnes maze;
2) Animals trained on the Barnes maze, receiving one single infusion of artificial cerebrospinal fluid 5min before commencement of the first trial on the first day; and
3) Animals trained on the Barnes maze, receiving one single dose of 100pmol Nle-Ang IV or LVVYPWTQRF (SEQ ID NO:2) 5min before the commencement of the first trial on the first day.

All rats are injected with bromodeoxyuracil (BrdU; 50mg/kg) twice daily for 3 days. The two groups of rats subjected to the behavioural paradigm are tested for 3 days on the Barnes maze, commencing the day of the first BrdU injection. During this time most of the rats which receive the peptide treatment are expected to achieve learner criteria, whereas the rats treated with artificial cerebrospinal fluid do not. The animals are perfused intracardially with 4% paraformaldehyde in phosphate buffered saline 24h after the last BrdU injection. The brains of these animals are removed and processed for BrdU immunofluorescence using a mouse anti-BrdU primary antibody and detected with an anti-mouse secondary antibody conjugated with FITC. The number of BrdU-labelled cells are determined on at least 24 sections (14µm thick) of the rat hippocampus in the rostro-caudal extent.

To determine if the increase in neurogenesis induced by Ang IV or LVVYPWTQRF (SEQ ID NO:2) is due to prolonging the half-life of insulin or insulin-like growth factor, this experiment is repeated in the presence of a specific antibodies raised against insulin or IGF.

### EXAMPLE 26 ROLE OF AT₄ RECEPTOR/IRAP IN TISSUE DEVELOPMENT AND REGENERATION

AT₄ receptor ligands have been shown to induce cellular proliferation in cardiac fibroblasts and neuronally-derived immortalised cell lines. This suggests that AT₄ receptor/IRAP may be abundantly expressed during development, and may have an important role in embryonic development. We investigated the expression of AT₄ receptors/IRAP in embryonic and neonatal mice, and compared the levels of expression with those of adult mice. Quantitative *in vitro* receptor autoradiography and *in situ* hybridisation histochemistry was used to determine the levels of AT₄ receptor/IRAP protein and mRNA.

Embryonic day 13,15,17, neonatal day 1,7,21 and adult mice were killed by isofluorane inhalation. The embryos were embedded in mounting medium and snap-frozen whole, whereas in the neonatal and adult mice, tissues were dissected out and frozen in isopentane chilled to -40°C on dry ice. For the determination of AT₄ receptor/IRAP protein, 10µ frozen sections were cut, thaw-mounted on to gelatine-coated slides, and dried for 2h under reduced pressure. The sections were then rinsed in buffer (pH 7.4) containing 50mM Tris, 5mM EDTA, 150mM NaCl for 30min at room temperature. This was followed by a 2h incubation in the buffer described above to which had been added 100µM PMSF, 20µM bestatin, 100µM phenanthroline, 0.1% BSA and 130pM ¹²⁵I-Nle¹ Ang IV. The sections were then washed four times in Tris-buffered saline at 0°C for 2min each, dried rapidly and exposed to X-ray film.

For the determination of AT₄ receptor/IRAP mRNA, 10µ frozen sections were cut, thaw-mounted onto silane-coated slides and stored at -20°C. Eight oligonucleotides, four in the anti-sense orientation and four in the sense orientation, were prepared from different coding regions of the AT₄ receptor/IRAP gene. The oligonucleotides were 3' end labelled with ³³P-dATP using terminal d-transferase and purified on a P6 column. The mouse sections were hybridized with 1 x 10⁶ cpm of labelled oligonucleotide in a 100ml total volume of 50% formamide, 4xSSC, 1xDenhardt's solution, 2% sarcosyl, 20mM sodium phosphate buffer (pH7.0), 10% dextran sulphate, 50µg/ml herring sperm DNA and 0.2mM dithiothreitol. After a 16h hybridization period at 42°C, the sections were washed four times in 1xSSC at 55°C, rinsed in distilled water and dehydrated through increasing alcohol. The sections were then exposed to X-ray film. The results are shown in Figure 8.

The highest level of AT₄ receptor/IRAP protein was found in developing bone. High levels of the protein were also detected in the heart, kidney, thymus, pituitary and the subventricular zone of the brain (Figure 8). These results suggest that the AT₄ receptor/IRAP system might play an important role in the development of these tissues. The distribution of AT₄ receptor/IRAP in the foetal and neonatal mice closely resemble the distribution of GLUT4 mRNA (Venucci et al., 1999). This suggests that AT₄ receptor/IRAP may have an autoregulatory role in the transport of glucose into cells during development, as is the case in adults.

### EXAMPLE 27 LVV-HAEMORPHIN 7 AND ANG IV INDUCTION OF DNA SYNTHESIS IN NEURONAL CELL LINES

Ang IV has been reported to induce DNA synthesis in rabbit and rat cardiac fibroblasts and in rat anterior pituitary cells. Moreover, in bovine coronary venular endothelial cells, Ang IV enhanced ³H-thymidine incorporation in response to stimulation with basic fibroblast growth factor, although Ang IV alone was a very weak stimulant.

The effect of LVV-haemorphin 7 and Ang IV on cellular proliferation was investigated in cells of the neuronally-derived immortalised cell lines NG108-15 and SK-N-MC, as appreciable amounts of AT₄ receptors/IRAP were shown to be present in these cell lines. NG108-15 is a rat-mouse hybrid cell line consisting of both neurones and glia, whereas the SKN-MC cells are neuroepithelial in origin. SK-N-MC cells are known to display.cholinergic properties, such as the expression of choline acetyltransferase and muscarinic receptors.

NG108-15 and SK-N-MC cells were cultured in DMEM supplemented with 10% heat-inactivated FCS, 2% HAT supplement, and 100U/ml penicillin/streptomycin. Cells were routinely used between passages 20 and 30.

Cells were plated at a density of 3x10⁴ cells/well in 24 well Falcon plates that had been coated with 0.1mg/ml poly-L-lysine hydrobromide. The cells were allowed to adhere overnight, and then serum deprived for 48h before being treated with LVVYPWTQRF (SEQ ID NO:2) and/or Ang IV (10⁻¹⁰ to 10⁻⁶ M) for 24h in medium with or without serum. Five hours before the end of the experiment 1µCi of ³H-thymidine was added to each well. At the completion of the incubation, cells were washed twice with 1ml of PBS at 4°C, followed by 5% trichloroacetic acid at 4°C for 30min, which was aspirated before solubilizing the cells in 1ml 0.25 M NaOH and 0.1% SDS for 30min at 37°C. Samples were then collected into 5ml of Ready Value scintillation fluid and ³H measured in a liquid scintillation counter. The results are summarised in Figure 21.

In the NG108-15 cells, LVVYPWTQRF (SEQ ID NO:2) at concentrations of 10⁻¹⁰ to 10⁻⁸ M stimulated ³H-thymidine incorporation in a dose-dependent manner to between 140 to 170% of control levels (p<0.05). In contrast, at concentrations of 10⁻¹⁰ to 10⁻⁶ M, Ang IV did not stimulate DNA synthesis in these cells. The ability of LVVYPWTQRF (SEQ ID NO:2) to increase ³H-thymidine incorporation was completely abolished by concomitant presence of 10⁻⁶ M Ang IV, in which situation the result was not significantly different from control (p=0.337). Ang IV dose-dependently inhibited the effect of 10⁻⁶ M LVVYPWTQRF (SEQ ID NO:2) in stimulating ³H-thymidine incorporation. At 10⁻¹⁰ M, Ang IV was inactive, whereas increasing concentrations of Ang IV from 10⁻⁹ to 10⁻⁷ M progressively inhibited ³H-thymidine incorporation.

Treatment of SK-N-MC cells with increasing concentrations of Ang IV (10⁻¹⁰ - 10⁻⁷ M) significantly increased [³H]-thymidine uptake, by up to 72% above control levels. LVVYPWTQRF (SEQ ID NO:2) also significantly stimulated [³H]-thymidine uptake at 10⁻¹⁰-10⁻⁶ M, by up to 81% above control levels, with maximal effects observed at 10⁻⁹ to 10⁻⁷ M LVVYPWTQRF (SEQ ID NO:2) (P<0.001). This effect was different to that observed in NG108-15 cells, where the stimulatory effect of LVVYPWTQRF (SEQ ID NO:2) was attenuated by Ang IV, with Ang IV alone not having any effect on DNA synthesis. In SK-N-MC cells, both peptides stimulated DNA synthesis in a dose-dependent manner.

The lack of effect of Ang IV on ³H-thymidine incorporation in NG108-15 cells was not due to the degradation of the peptide. In a parallel study, using conditions similar to that of the DNA synthesis experiments, the integrity of the peptide was determined by HPLC. Ang IV did undergo some degradation, but this was insufficient to explain its lack of activity.

### EXAMPLE 28 LVVYPWTQRF (SEQ ID NO:2) AND ANG IV INHIBITS NEURITE OUTGROWTH IN CHICK SYMPATHETIC NEURONES

The wide distribution of the AT₄ receptors/IRAP in the brain suggests important roles for this enzyme in the brain in the processing of several neuropeptides. Moreover, the distribution of AT₄ receptors/IRAP closely correlated with the distribution of cholinergic neurones and their processes. Acetylcholine, the neurotransmitter in cholinergic neurones, is known to inhibit neurite outgrowth in embryonic chick ciliary ganglion cells. We have examined whether either of the AT₄ receptor ligands, Ang IV and LVVYPWTQRF (SEQ ID NO:2), had a trophic effect in the central nervous system, and whether the effect paralleled that of acetylcholine. We examined whether either peptide exerted any effect on neurite outgrowth from cultured chick sympathetic neurones.

Sympathetic ganglia from embryonic day 11 chicks were dissociated with trypsin/versene and were cultured in 24 well plates in DMEM and Ham's 12 medium which contained 1% insulin-tranferrin-selenium-X growth supplement, 100 mM putrescine, 1.67mg/ml prostaglandin F2α, 6.67ng/ml progesterone, and 5ng/ml nerve growth factor. Neurones were allowed to adhere to the wells (approximately 2h) before being treated with the peptides. Dose response curves were performed over the concentration range 10⁻¹¹ to 10⁻⁵M. Culture dishes were coated with 0.1mg/ml poly-L-lysine and washed with PBS before receiving another coating of laminin (10µg/ml).

At the conclusion of the incubation, the culture medium was removed from the wells, the neurones fixed with 2.5% glutaraldehyde in phosphate-buffered saline for 20min, and examined under phase contrast microscope attached to an MD30 Plus Image Analysis software system. The length of neurites (longer than 50µm) of every neurone examined was measured. A minimum of 40 neurite measurements was taken per treatment group, and each dose of peptide was repeated at least three times. At the conclusion of the experiment, the viability of the cells was confirmed by exclusion of 0.1% aniline blue. The results are shown in Figure 22.

In cultures of embryonic chick sympathetic neurones, Ang IV and LVVYPWTQRF (SEQ ID NO:2) inhibited neurite outgrowth in a dose dependent manner, with maximal effect achieved at concentrations between 10⁻⁹ to 10⁻⁸ M. The maximal extent of inhibition of neurite outgrowth was approximately 30%, which was comparable to the effect achieved by acetylcholine. These results suggest a role for AT₄ receptors/IRAP in remodelling of neurones.

### EXAMPLE 28 ROLE OF AT₄ RECEPTOR/IRAP IN BONE DEVELOPMENT

As described in Example 26, we also found a high concentration of AT₄ receptor/IRAP in the developing bone of E13, E15, E17 mouse embryos. IGF-I receptors have been shown to co-localize with GLUT4 in chondroblastic and hypertrophic cells of mouse bone growth centres. Moreover, in the streptozotocin-induced diabetic mouse model, there are severe histological changes in the mandibular condyles and humeral growth plate which are associated with decreases in GLUT4 and IGF-I and insulin receptors. We therefore consider that AT₄ receptor/IRAP co-localizes with GLUT4, and plays an important role in the regulation of IGF-I in regulating early bone growth.

### EXAMPLE 30 EFFECT OF AT₄ RECEPTOR/IRAP LIGANDS ON BRAIN DAMAGE

Activated astrocytes which are glial fibrillary acidic protein positive are involved in neuronal tissue remodelling after damage (Bovolenta et al 1992). Injury-evoked plasticity is similar to events observed in the developing central nervous system (Schwartz 1992). IGF-I is a growth factor which is not only important in normal growth and development; there is strong evidence to suggest that it has an important role in neurodegenerative conditions in adult. Injury to the brain results in an up-regulation of IGFs, their receptors and binding proteins. Application of exogenous IGF-I protects the brain from hypoxic and ischemic damage. In the light of our findings of high levels of AT₄ receptor/IRAP expression in the developing brain, as shown in Figure 23, and an induction of AT₄ receptor/IRAP expression in activated astrocytes in mechanically-damaged spinal cord, as shown in Figure 24, we hypothesise that modulators of AT₄ receptor/IRAP activity will have a neuroprotective role in different animal models of damage.

To test this hypothesis, male Sprague-Dawley rats are anaesthetized with isofluorane in O₂ and indwelling intracerebroventricular cannulae are implanted. The cannula are connected to Alzet minipumps containing artificial cerebrospinal fluid which are implanted subcutaneously in the dorsal neck region. Seven days later, the rats are re-anaesthetised with halothane in O₂, and focal cerebral ischemia induced by permanent occlusion of the left, middle cerebral artery proximal to the lenticulostriate branch, by electrocoagulation. During the surgery, the Alzet minipumps containing artificial cerebrospinal fluid are replaced with ones containing either:
1) Artificial cerebrospinal fluid; or
2) 10 or 100pmol/h Nle-Ang IV; or
3) 10 or 100pmol/h LVVYPWTQRF (SEQ ID NO:2).

Two or seven days after induction of the cerebral ischemia, the animals are killed, the brains removed, and 50µm sections are taken and stained with 2% tri-phenyltetrazolium chloride. The volumes of the infarcts are determined on the sections from peptide-treated animals, and compared to those in artificial cerebrospinal fluid controls.

### EXAMPLE 31 ROLE OF IRAP/AT₄ RECEPTOR IN BLOOD VESSELS

The vascularisation of tissues during development occurs by the process of angiogenesis, in which endothelial cells from existing capillaries form new capillaries by sprouting or by splitting from their tissue of origin. Little angiogenesis occurs in adult tissues, except during wound healing, during the female reproductive cycle and in certain pathological states such as cancer. Pathological angiogenesis involves the persistent proliferation of endothelial cells, as occurs in proliferative retinopathy, rheumatoid arthritis, psoriasis and tumour growth. Angiogenesis is also essential to the growth of solid tumours.

The observed occurrence of AT₄ receptor/IRAP in developing blood vessels near the site of a wound, illustrated in Figure 24, suggests that AT₄ receptor/IRAP has a role in angiogenesis during wound healing, and possibly in the pathological conditions mentioned above. If this is so, the potential exists to use modulators of IRAP/AT₄ to interfere with angiogenesis and thereby limit tumour invasion, and/or to treat other conditions in which pathogenic angiogenesis is a feature. Peptidases are known to have many roles in angiogenesis. Degradation of extracellular matrix (ECM) by peptidases allows the infiltration of endothelial cells from existing blood vessels.

ECM degradation is a tightly controlled process, and peptidases also play roles in its regulation. For instance, ECM-degrading proteinases mobilise growth factors such as fibroblast growth factor (FGF)from the ECM, which stimulates expression of collagenase and plasminogen activator by microvascular endothelial cells. ECM-degrading proteinases also modulate activation of latent TGF-β1 in the ECM, which counteracts the stimulatory effects of FGF on vascular endothelial cells.

A number of experimental systems are used to test the role of AT₄ receptor/IRAP in angiogenesis. When endothelial cells are grown on a gel formed of reconstituted basement membrane proteins (Matrigel), they aggregate and organise into tubule-like structures (Kubota et al., 1988). The ability of peptides that bind to AT₄ receptor/IRAP (e.g. Ang IV, LVVYPWTQRF (SEQ ID NO:2), KYLPGPLQ (SEQ ID NO:3) and divalina1-Ang IV) to modulate this process is assessed by culturing human umbilical vein endothelial (HUVE) cells or human dermal microvascular cells on Matrigel, with or without pre-incubation of the cells with IRAP/ AT₄ receptor -binding peptide. Alternatively the peptide is incorporated into the gel matrix. The role of AT₄ receptor/IRAP in angiogenesis is further assessed by incorporating antibodies to AT₄ receptor/IRAP into the matrigel before application of HUVE cells or HDME cells.

The role of IRAP/AT₄ in tumour angiogenesis is initially investigated using receptor autoradiography with [125I]-Nle-Ang IV to examine the distribution of IRAP/AT₄ in the microvasculature in sections of human tumour tissue. Next, the ability of IRAP/AT₄ ligands to modulate tumour angiogenesis is assessed in an *in vivo* model in which C3L5 cells,a highly metastatic murine mammary adenocarcinoma cell line, are suspended in growth factor-reduced Matrigel and implanted subcutaneously into mice. Growth factor-reduced Matrigel alone does not stimulate angiogenesis (Jadeski & Lala, 1999), but angiogenesis occurs in implants containing tumour cells. IRAP/AT₄ ligands are administered via osmotic minipumps over 14 days, after which their effects on angiogenesis within the implants are assessed.

### EXAMPLE 32 UPREGULATION OF AT₄ RECEPTOR/IRAP IN ATHEROSCLEROTIC BLOOD VESSELS

Ang IV binds to AT₄ receptors in blood vessels to induce vasodilatation and proliferation of cultured bovine endothelial cells. Topical application of Ang IV caused vasodilatation of pial arterioles, and infusion of Ang IV into the rat middle cerebral artery and renal artery significantly increased blood flow. Ang IV significantly but modestly increased DNA synthesis in cultured bovine endothelial cells, and synergistically enhanced basic fibroblast growth factor-stimulated cellular proliferation.

Twelve week old male New Zealand White rabbits were anaesthetised with propofol (10 mg/kg, intravenously) followed by halothane, administered via an endo-tracheal tube. A 2F Fogarty embolectomy catheter was inserted through an arteriotomy of a branch of the external right carotid artery and extended to the aortic arch. The catheter was inflated and withdrawn to the bifurcation of the common carotid artery in order to de-endothelialize the vessel. This was repeated three times to ensure complete de-endothelialization. The uninjured left carotid arteries were used as control vessels. Three rabbits per group at 1 day and 1 week post surgery, and 4 rabbits per group at 4 weeks and 20 weeks post surgery, were killed with a lethal dose of pentobarbitone and left and right carotid arteries were removed and rapidly frozen in isopentane cooled to -40°C.

Carotid arteries were sectioned at 20µm thickness. Sections were preincubated in an isotonic buffer, 50mM Tris-HCl buffer, pH 7.4, 150mM NaCl and 5mM EDTA for 30min at 22°C and then incubated in 50mM Tris-HCl, pH 7.4, 150mM NaCl, 5mM EDTA, 100µM phenylmethyl-sulfonyl fluoride (Sigma, Mo., U.S.A.), 20µM bestatin (Peninsula Laboratories, Ca., U.S.A.) and 0.1% bovine serum albumin, containing approximately 200pM [¹²⁵I] Ang IV, for 2h at 22°C. Non-specific binding was determined in the presence of 1µM Ang IV in adjacent sections. Following the incubation, the sections were given 3 x 2min washes in the isotonic buffer, at 4°C, air dried, and exposed to X-ray film for 3.5 weeks, together with a set of radioactive standards. After exposure, the sections were counterstained with haematoxylin and eosin for histological examination.

Images were quantified on an MCID image analysis system (Image Research, Ontario Canada), in which the pixel densities on the X-ray films were converted to radioactive units (dpm/mm²) using the radioactive standards. The vessel wall thickness was measured using the MCID system. The results are shown in Figures 25 and 26.

In normal rabbit carotid arteries, AT₄ receptors were detected in vascular smooth muscle cells and in the vasa vasorum of the adventitia, as shown in Figure 25, left-hand panel. Very low receptor levels were observed in the endothelial cells.

Within the first week following endothelial denudation of the carotid artery by balloon catheter, AT₄ receptor/IRAP in the media/neointima increased to approximately 150% of control tissue, as shown in Figures 26A and 26B. AT₄ receptor/IRAP further increased in the media/neointima and re-endothelialized cell layer to 223% at 20 weeks after injury (Figures 26C and 26D).

In view of the known trophic effects of Ang IV, the elevated expression of IRAP/AT₄ receptors in both the neointima and media of arteries following balloon injury to the endothelium, suggests a role for the IRAP/AT₄ receptor in the adaptive response and remodelling of the vascular wall following damage.

### EXAMPLE 33 Elucidation of factors which translocate AT₄ receptor/IRAP to the cell surface in neuronal cells

In insulin-responsive cells in the basal state, a major proportion of AT₄ receptor/IRAP and GLUT4 is co-localised inside the cell in specialised post-endosomal vesicles, which are distinct from constitutively recycling endosomes, and also in the trans-Golgi network. Both GLUT4 and AT₄ receptor/IRAP rapidly translocate to the plasma membrane in response to insulin stimulation; GLUT4 to mediate insulin-stimulated glucose uptake(Garza et al, 2000).

This process involves the formation of vesicles containing both GLUT4 and AT₄ receptor/IRAP, which translocate to the cell surface, and fuse with the plasma membrane by a mechanism which in part parallels synaptic vesicle trafficking and docking in the regulation of neurotransmitter release in the brain (Cheatham 2000). The molecular mechanism/signalling cascade regulating the trafficking of GLUT4/ AT₄ receptor/IRAP vesicles has not been fully elucidated. In addition to insulin, osmotic shock, guanosine 5'-O-(3-thiotriphosphate) (GTPγS) and adrenergic stimulation also result in the translocation of AT₄ receptor/IRAP and GLUT4 to the cell surface in adipocytes and/or skeletal muscle cells. In human vascular endothelial cells, activation of the oxytocin receptor by oxytocin, a substrate for AT₄ receptor/IRAP, was demonstrated to translocate IRAP to the cell surface (Nakamura et al, 2000).

We have established stably transfected cell lines of both SK-N-MC and SN56 cells expressing A_{T4} receptor/IRAP with a c-Myc tag at the extracellular carboxy terminal. These cell lines enable us to rapidly and quantitatively determine the factors or compounds which result in the translocation of IRAP to the cell surface. Serum-deprived cells are incubated with different candidates, such as insulin, IGF-I, neurotensin, or AT₄ receptor/IRAP substrates, or under depolarizing conditions, and the level of AT₄ receptor/IRAP at the plasma membrane measured after 30 min using an in-house ELISA assay. After positive factors have been identified, time-course studies are performed.

The results using this system are verified by performing subcellular fractionation using sucrose gradient centrifugation, followed by Western Blot analysis of AT₄ receptor/IRAP in stimulated and unstimulated cells. The first set of experiments measures endogenous AT₄ receptor/IRAP in untransfected cells, to verify that the results obtained are not an artefact of the transfection system used. In the second series of experiments we determine the subcellular vesicles which are recruited to the plasma membrane following stimulation. Light density microsomes from stimulated and non-stimulated cells are immunoadsorbed with antibodies to the different vesicle marker proteins, and the level of AT₄ receptor/IRAP for each vesicle type determined by Western Blot analysis.

We have demonstrated that serum stimulates the translocation of IRAP to the cell surface in both SK-N-MC and SN56 cells. We are identifying the factors, compounds or conditions which will translocate IRAP to the cell surface.

References cited herein are listed on the following pages.

### REFERENCES

Barnes, C. (1979). Memory deficits associated with senescence: a neurophysiological and behavioral study in the rat. J Comp Physiol Psychol. 93, 74-104.
Bondy, C. (1991). Transient IGF-I gene expression during the maturation of functionally related central projection neurons. J Neurosci. 11, 3442-55.
Bovolenta P, W. F., Nieto-Sampedro M. (1992). CNS glial scar tissue: a source of molecules which inhibit central neurite outgrowth. Prog Brain Res. 94, 367-379.
Braszko, J. J., Kupryszewski, G., Witczuk, B., and Wisniewski, K. (1988). Angiotensin II-(3-8)-hexapeptide affects motor activity, performance of passive avoidance and a conditioned avoidance response in rats. Neuroscience 27, 777-83.
Chai, S. Y., Bastias, M. A., Clune, E. F., Matsacos, D. J., Mustafa, T., Lee, J. H., McDowall, S. G., Mendelsohn, F. A., Albiston, A. L., and Paxinos, G. (2000). Distribution of angiotensin IV binding sites (AT(4) receptor) in the human forebrain, midbrain and pons as visualised by in vitro receptor autoradiography. J Chem Neuroanat 20, 339-348.
Cheatham, B. (2000). GLUT4 and company: SNAREing roles in insulin-regulated glucose uptake. Trends Endocrinol Metab 11, 356-61.
Chi, N. W., and Lodish, H. F. (2000). Tankyrase is a golgi-associated mitogen-activated protein kinase substrate that interacts with IRAP in GLUT4 vesicles. J Biol Chem 275, 38437-44.
Chorev, M. and Goodman, M. (1993). Acc. Chem. Res., 26, p266-273.
Delorenzi, A., and Maldonado, H. (1999). Memory enhancement by the angiotensinergic system in the crab Chasmagnathus is mediated by endogenous angiotensin II. Neurosci Lett 266, 1-4.
Eng, J., McCormack, AL, Yates, JR 3rd (1994). An approach to correlate tandem mass spectral data of peptides with amino acid sequences in a protein database. J. Am. Soc. Mass. Spectrom. 1994 5, 976-989.
Foster LJ, K. A. (2000). Mechanism and regulation of GLUT-4 vesicle fusion in muscle and fat cells. Am J Physiol 279, C877-90.
Gallop, M.A., Barrett, R.W., Dower, W.J., Fodor, S.P.A. and Gordon, E.M. (1994). J. Med. Chem., 37, p1233-1251.
Garza, L. A., and Birnbaum, M. J. (2000). Insulin-responsive aminopeptidase trafficking in 3T3-L1 adipocytes. J Biol Chem 275, 2560-7.
Henderson, AS and Jorm, AF. (1998). Aged and Community Care Service Development and Evaluation Report No. 35. Canberra: AGPS.
Herbst, J. J., Ross, S. A., Scott, H. M., Bobin, S. A., Morris, N. J., Lienhard, G. E., and Keller, S. R. (1997). Insulin stimulates cell surface aminopeptidase activity toward vasopressin in adipocytes. Am J Physiol 272, E600-6.
Holman, G. D., and Sandoval, I. V. (2001). Moving the insulin-regulated glucose transporter GLUT4 into and out of storage. Trends Cell Biol 11, 173-9.
Jadeski LC, L. P. (1999). Nitric oxide synthase inhibition by N(G)-nitro-L-arginine methyl ester inhibits tumor-induced angiogenesis in mammary tumors. Am J Pathol. 155, 1381-90.
Kopf, S. R., Buchholzer, M. L., Hilgert, M., Loffelholz, K., and Klein, J. (2001). Glucose plus choline improve passive avoidance behaviour and increase hippocampal acetylcholine release in mice. Neuroscience 103, 365-71.
Kubota Y, K. H., Martin GR, Lawley TJ. (1988). Role of laminin and basement membrane in the morphological differentiation of human endothelial cells into capillary-like structures.
   J Cell Biol. 107, 1589-98.
Matsumoto, H., Rogi, T., Yamashiro, K., Kodama, S., Tsuruoka, N., Hattori, A., Takio, K., Mizutani, S., and Tsujimoto, M. (2000). Characterization of a recombinant soluble form of human placental leucine aminopeptidase/oxytocinase expressed in Chinese hamster ovary cells. Eur J Biochem 267, 46-52.
Miller-Wing, A. V., Hanesworth, J. M., Sardinia, M. F., Hall, K. L., Wright, J. W., Speth, R. C., Grove, K. L., and Harding, J. W. (1993). Central angiotensin IV binding sites: distribution and specificity in guinea pig brain. J Pharmacol Exp Ther 266, 1718-26.
Moeller, I., Chai, S. Y., Oldfield, B. J., McKinley, M. J., Casley, D., and Mendelsohn, F. A. (1995). Localization of angiotensin IV binding sites to motor and sensory neurons in the sheep spinal cord and hindbrain. Brain Res 701, 301-6.
Moeller, I., Lew, R. A., Mendelsohn, F. A., Smith, A. I., Brennan, M. E., Tetaz, T. J., and Chai, S. Y. (1997). The globin fragment LVV-hemorphin-7 is an endogenous ligand for the AT4 receptor in the brain. J Neurochem 68, 2530-7.
Moeller, I., Paxinos, G., Mendelsohn, F. A., Aldred, G. P., Casley, D., and Chai, S. Y. (1996). Distribution of AT4 receptors in the Macaca fascicularis brain. Brain Res 712, 307-24.
Mortiz, R., Reid, GE, Ward, LD, Simpson, RJ (1994). Methods 6, 213-226.
O'Kusky, J. R., Ye, P., and D'Ercole, A. J. (2000). Insulin-like growth factor-I promotes neurogenesis and synaptogenesis in the hippocampal dentate gyrus during postnatal development. J Neurosci 20, 8435-42.
Olson, G.L., Bolin, D.R., Bonner, M.P., Bos, M., Cook, C.M., Fry, D.C., Graves,B.J., Hatada, M., Hill, D.E., Kahn, M., Madison, V.S., Rusiecki, V.K., Sarabu, R., Sepiwall, J., Vincent, G.P. and Voss, M.E. (1993). J. Med. Chem., 36, p3039-3049.
Pederson, E. S., Harding, J. W., and Wright, J. W. (1998) . Attenuation of scopolamine-induced spatial learning impairments by an angiotensin IV analog. Regul Pept 74, 97-103.
Pessin, J. E., Thurmond, D. C., Elmendorf, J. S., Coker, K. J., and Okada, S. (1999). Molecular basis of insulin-stimulated GLUT4 vesicle trafficking. Location! Location! Location! J Biol Chem 274, 2593-6.
Rasmussen, T. E., Pedraza-Diaz, S., Hardre, R., Laustsen, P. G., Carrion, A. G., and Kristensen, T. (2000). Structure of the human oxytocinase/insulin-regulated aminopeptidase gene and localization to chromosome 5q21. Eur J Biochem 267, 2297-306.
Roberts, K. A., Krebs, L. T., Kramar, E. A., Shaffer, M. J., Harding, J. W., and Wright, J. W. (1995). Autoradiographic identification of brain angiotensin IV binding sites and differential c-Fos expression following intracerebroventricular injection of angiotensin II and IV in rats. Brain Res 682, 13-21.
Roepstorff P, F. J. (1984). Proposal for a common nomenclature for sequence ions in mass spectra of peptides. Biomed Mass Spectrom. 11, 601.
Rogi, T., Tsujimoto, M., Nakazato, H., Mizutani, S., and Tomoda, Y. (1996). Human placental leucine aminopeptidase/oxytocinase. A new member of type II membrane-spanning zinc metallopeptidase family. J Biol Chem 271, 56-61.
Ross, S. A., Keller, S. R., and Lienhard, G. E. (1998). Increased intracellular sequestration of the insulin-regulated aminopeptidase upon differentiation of 3T3-L1 cells. Biochem J 330, 1003-8.
Schwartz, J. (1992). Neurotransmitters as neurotrophic factors: a new set of functions. Int Rev Neurobiol. 34, 1-23.
Shevchenko A, W. M., Vorm O, Mann M. (1996). Mass spectrometric sequencing of proteins silver-stained polyacrylamide gels. Anal Chem. 68, 850-8.
Simpson, F., Whitehead, J. P., and James, D. E. (2001). GLUT4--at the cross roads between membrane trafficking and signal transduction. Traffic 2, 2-11.
Swanson, G. N., Hanesworth, J. M., Sardinia, M. F., Coleman, J. K., Wright, J. W., Hall, K. L., Miller-Wing, A. V., Stobb, J. W., Cook, V. I., Harding, E. C., and et al. (1992). Discovery of a distinct binding site for angiotensin II (3-8), a putative angiotensin IV receptor. Regul Pept 40, 409-19.
Vannucci, S. J., Rutherford, T., Wilkie, M. B., Simpson, I. A., and Lauder, J. M. (1999). Prenatal expression of the GLUT4 glucose transporter in the mouse.
Waters, S. B., D'Auria, M., Martin, S. S., Nguyen, C., Kozma, L. M., and Luskey, K. L. (1997). The amino terminus of insulin-responsive aminopeptidase causes Glut4 translocation in 3T3-L1 adipocytes. J Biol Chem 272, 23323-7.
Wickelgren, I. (1998). Tracking insulin to the mind. Science 280, 517-9.
Wright, J. W., Clemens, J. A., Panetta, J. A., Smalstig, E. B., Weatherly, L. A., Kramar, E. A., Pederson, E. S., Mungall, B. H., and Harding, J. W. (1996). Effects of LY231617 and angiotensin IV on ischemia-induced deficits in circular water maze and passive avoidance performance in rats. Brain Res 717, 1-11.
Wright, J. W., Miller-Wing, A. V., Shaffer, M. J., Higginson, C., Wright, D. E., Hanesworth, J. M., and Harding, J. W. (1993). Angiotensin II(3-8) (ANG IV) hippocampal binding: potential role in the facilitation of memory. Brain Res Bull 32, 497-502.
Wright, J. W., Stubley, L., Pederson, E. S., Kramar, E. A., Hanesworth, J. M., and Harding, J. W. (1999). Contributions of the brain angiotensin IV-AT4 receptor subtype system to spatial learning. J Neurosci 19, 3952-61.
Zhang, J. (1998). Characterization and purification of the bovine adrenal angiotensin IV receptor using 125I benzoylphenyalanine-angiotensin IV as a specific photolabel. Journal of pharmacology and experimental therapeutics 287, 416-424.
Zhang, J. H., Hanesworth, J. M., Sardinia, M. F., Alt, J. A., Wright, J. W., and Harding, J. W. (1999). Structural analysis of angiotensin IV receptor (AT4) from selected bovine tissues. J Pharmacol Exp Ther 289, 1075-83.
Zuker, M. (1989).
   On finding all suboptimal foldings of an RNA molecule. 244, 48-52.

## Claims

1. A method of *in vitro* screening for compounds that have the ability to enhance memory and learning, comprising assessing a candidate compound's ability to modulate an enzymatic activity of the AT₄ receptor, wherein the enzymatic activity of the AT₄ receptor is aminopeptidase activity.

## Patentansprüche

1. Verfahren für das In-Vitro-Screening für Verbindungen, die die Fähigkeit haben, das Gedächtnis und das Lernen zu steigern, umfassend das Einschätzen der Fähigkeit einer Kandidatenverbindung, eine enzymatische Aktivität des AT₄-Rezeptors zu modulieren, wobei die enzymatische Aktivität des AT₄-Rezeptors eine Aminopeptidase-Aktivität ist.

## Revendications

1. Procédé de criblage *in vitro* pour des composés qui ont la capacité d'améliorer la mémoire et l'apprentissage, comportant l'estimation d'une capacité d'un composé candidat à moduler une activité enzymatique du récepteur AT₄, dans lequel l'activité enzymatique du récepteur AT₄ est une activité aminopeptidase.
